(19) Europäisches Patentamt
European Patent Office
Office européen des brevets

(11) Numéro de publication : **0 105 881**
**B1**

(12) **FASCICULE DE BREVET EUROPÉEN**

(45) Date de publication du fascicule du brevet : **23.12.87**

(51) Int. Cl.[4] : **C 07 D405/06**, A 61 K 31/445

(21) Numéro de dépôt : **83901083.2**

(22) Date de dépôt : **08.04.83**

(86) Numéro de dépôt international : **PCT/FR 83/00066**

(87) Numéro de publication internationale : **WO/8303607 (27.10.83 Gazette 83/25)**

(54) **NOUVELLES CYANOGUANIDINES, LEUR PROCEDE D'OBTENTION ET LES COMPOSITIONS PHARMACEUTIQUES EN RENFERMANT.**

(30) Priorité : **08.04.82 FR 8206128**

(43) Date de publication de la demande : **25.04.84 Bulletin 84/17**

(45) Mention de la délivrance du brevet : **23.12.87 Bulletin 87/52**

(84) Etats contractants désignés : **AT BE CH DE FR GB LI LU NL SE**

(56) Documents cités : **EP-A- 0 004 358**

(73) Titulaire : **BOUCHARA S.A.**
**47, rue de Bretagne**
**F-75003 Paris (FR)**

(72) Inventeur : **CORNU, Pierre-Jean**
**100, avenue Kléber**
**F-75116 Paris (FR)**
Inventeur : **PERRIN, Claude**
**5, rue de l'Avenir**
**F-91400 Orsay (FR)**
Inventeur : **DUMAITRE, Bernard**
**24, rue Chemin Vert**
**F-93000 Bobigny (FR)**
Inventeur : **STREICHENBERGER, Gilles**
**30, boulevard du Château**
**F-92200 Neuilly sur Seine (FR)**

(74) Mandataire : **Burtin, Jean-François**
**Laboratoires Bouchara 8, rue Pastourelle**
**F-75003 Paris (FR)**

**EP 0 105 881 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet de nouveaux dérivés de la guanidine ainsi que leurs procédés d'obtention.

Elle a plus particulièrement pour objet de nouvelles N-cyano guanidines liées à un cycle pipéridine substitué.

Elle a spécifiquement pour objet des [(2,3 dihydrobenzo 1,4-dioxinyl-2) alcoyl] pipéridinyl-4 (N-cyano N'-R) guanidines de formule générale I

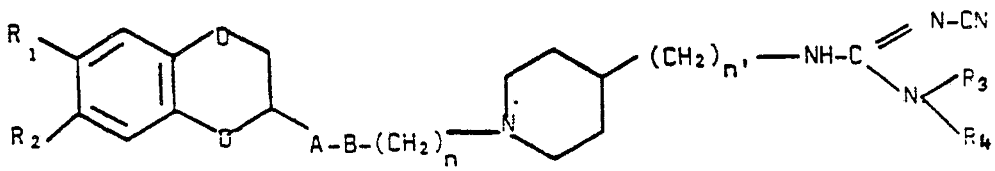

(I)

dans laquelle

$R_1$ et $R_2$ identiques ou différents, représentent de l'hydrogène, un radical alcoyle inférieur, un radical alcoxy inférieur, un halogène ou un radical trifluorométhyle

$R_3$ représente un radical alcoyle inférieur ayant de 1 à 6 atomes de carbone, un radical alcényle inférieur ayant de 2 à 4 atomes de carbone, un radical cycloalcoyle inférieur ayant de 3 à 7 atomes de carbone ou un radical hétérocyclanyle ayant 5, 6 ou 7 chaînons.

$R_4$ représente de l'hydrogène, un radical alcoyle inférieur ou le reste acyle d'un acide organique carboxylique ayant de 1 à 12 atomes de carbone

ou $R_3$ et $R_4$ forment ensemble le reste alcoylène d'un hétérocycle azoté comportant éventuellement un autre hétéroatome ayant de 5 à 7 maillons

n est égal à 0 ou 1

n' est égal à 0 ou à 1

et A représente $—CH_2—$ ou une liaison simple et B représente —CHOH— ou —C = O

ou bien A représente —CHOH— ou —C = O et B représente une liaison simple ou $—CH_2—$

ou bien A et B représentent $—CH_2—$ ou une liaison simple.

La formule générale I représente une des structures possibles des cyanoguanidines. En milieu acide, l'un quelconque des azotes de la fonction guanidine peut être protoné. Il en résulte que les composés selon l'invention peuvent exister sous les deux formes tautomères : imino-cyanoamine et amino-cyanoimine.

En outre, le groupe cyano peut se trouver d'un côté ou de l'autre du plan déterminé par la double liaison —C = N. Il en résulte une possibilité d'isomérie syn et anti.

Les formes tautomères et les formes isomères font partie de l'invention.

L'invention se rapporte aussi aux sels d'addition avec un acide minéral ou organique, de préférence un acide thérapeutiquement compatible, d'un composé de formule générale I.

L'invention se rapporte encore aux formes optiquement actives des composés de formule générale I ainsi qu'aux diastéréoisomères des composés de formule générale I.

Parmi les sels d'addition des composés de formule générale I, on pourra citer plus particulièrement les chlorhydrates, bromhydrates, sulfates, nitrates, phosphates, thiosulfates, les formiates, acétates, maléates, fumarates, benzoates, dichloro 2,6-benzoates, citrates, tartrates (méthoxy salicylates), 3,4,5-triméthoxy benzoates, les vanillates, les O-carbéthoxy syringoates, les naphtoates, les benzène sulfonates, les méthane sulfonates, les iséthionates, les nicotinates, les isonicotinates, les embonates et les glucose-phosphates.

Pour autant que l'invention soit concernée, un radical alcoyle inférieur est une chaîne hydrocarbonée ayant de 1 à 6 atomes de carbone en chaîne droite ou ramifiée, comme par exemple le méthyle, l'éthyle, l'isopropyle, le secbutyle, le terbutyle, le pentyle, le néopentyle et le n-hexyle.

Un radical alcoxy inférieur a de 1 à 6 atomes de carbone dans la chaîne alcoyle qui peut être droite ou ramifiée, comme un méthoxy, un éthoxy, un isopropoxy, un terbutoxy, ou un pentyloxy.

Un radical acyle est dérivé d'un acide organique carboxylique ayant de 1 à 12 atomes de carbone, comme par exemple un acide alcoyl carboxylique, un acide arylcarboxylique, un acide arylalcoyl carboxylique, un acide cycloalcoylcarboxylique ou un acide hétéroaryl carboxylique. On pourra citer à cet égard un acétyle, un butyryle, un benzoyle, un 3,4,5-triméthoxybenzoyle, un cyclopropylcarbonyle ou un nicotinoyle.

La signification des paramètres n et n' est importante et joue un rôle certain sur les propriétés pharmacologiques des composés de formule générale I. L'intensité ou la durée d'action des composés, selon l'invention, peut être modulée en modifiant la longueur de la chaîne carbonée de l'une ou l'autre partie de la molécule.

Lorsque $R_3$ et $R_4$ forment la chaîne alcoylène d'un hétérocycle azote, ils représentent avec l'atome d'azote auxquels ils sont liés un cycle pipéridinyl, pyrolidinyl, hexaméthylène imino ou heptaméthylène

imino ou bien lorsqu'ils incluent une autre hétéro-atome, un cycle hexahydropyrimidinyl, tétrahydrothiazinyl, morpholyl, pipérazinyl, N-alcoyl pipérazinyl, N-hydroxy alcoyl pipérazinyl, N-alcoxyalcoyl pipérazinyl ou N-acyloxyalcoyl pipérazinyl.

Parmi les composés de l'invention on citera plus particulièrement :

les composés de formule $I_A$

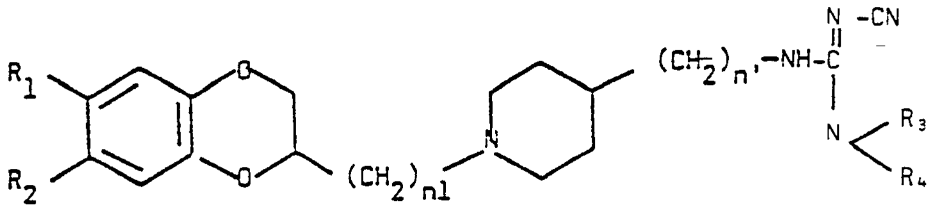

($I_A$)

dans laquelle $n_1$ représente un nombre entier variant de 0 à 3 et les substituants $R_1$, $R_2$, $R_3$, $R_4$ et n' ont les significations fournies précédemment, ainsi que les sels d'addition de ces composés avec un acide minéral ou organique ;

les composés de formule $I_B$

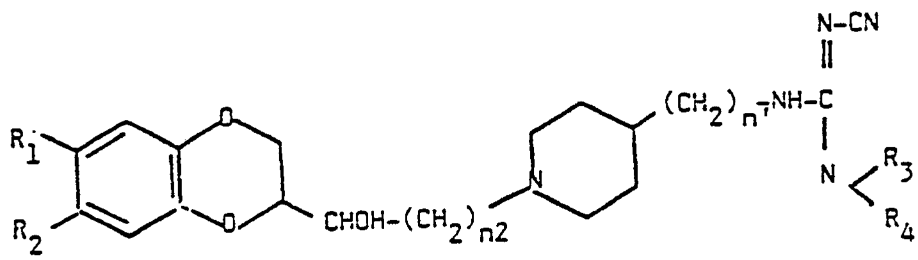

($I_B$)

dans laquelle $n_2$ représente un nombre entier variant de 0 à 2 et les substituants $R_1$, $R_2$, $R_3$, $R_4$ et n' ont les définitions précédentes, ainsi que les sels d'addition de ces composés avec un acide minéral ou organique ;

les composés de formule $I_C$

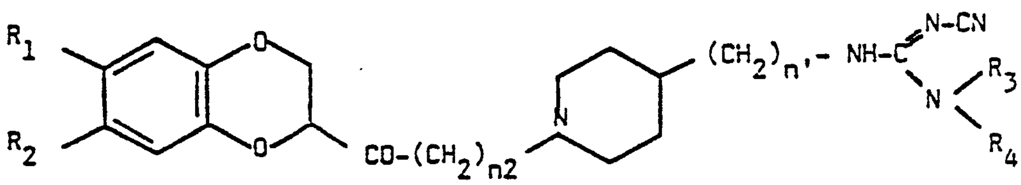

($I_C$)

dans laquelle $R_1$, $R_2$, $R_3$, $R_4$ n' et $n_2$ ont les significations fournies précédemment ainsi que les sels d'addition de ces composés avec un acide minéral ou organique et spécifiquement les composés suivants :

la 1-[(2,3-dihydro [4H] (1,4-benzodioxin-2 yl) méthyl] 4-(N-cyano N' méthyl guanidinyl) pipéridine

la 1-[2,3-dihydro [4H] (1,4-benzodioxin-2 yl) méthyl] 4-[(N-cyano N'-méthyl) guanidinyl méthyl] pipéridine

la 1-[(2,3-dihydro [4H] 1,4-benzodioxin-2 yl) méthyl] 4-[(N-cyano N'-allyl) guanidinyl méthyl] pipéridine

la 1-[(2,3-dihydro [4H] 1,4-benzodioxin-2 yl) méthyl] 4-[(N-cyano N'-(1-méthyl propyl) guanidinyl méthyl] pipéridine

la 1-[(2,3-dihydro [4H] 1,4-benzodioxin-2 yl) méthyl] 4-[(N-cyano N'-cyclopropyl) guanidinyl méthyl] pipéridine.

Les composés selon l'invention se distinguent par leurs propriétés pharmacologiques intéressantes et notamment par leurs propriétés anti-hypertensives et vaso-dilatatrices associées à une action sédative sur le système nerveux central. Du fait de leur haut niveau d'activité, les composés de formule générale I ou leurs sels d'addition trouvent un emploi en thérapeutique humaine ou animale comme principes actifs de médicaments destinés à combattre ou à réduire les effets de la maladie hypertensive.

A ces fins ils sont utilisés sous forme de compositions pharmaceutiques destinées à l'administration par voie parentérale, buccale, rectale ou sublinguale.

Les compositions pharmaceutiques renferment, comme principe actif, au moins un composé de formule générale I ou un de ses sels d'addition avec un acide minéral ou organique, en association ou en mélange avec un excipient ou un véhicule inerte, pharmaceutiquement acceptable.

On pourra citer comme formes d'administration préférées, les comprimés nus ou enrobés, les capsules, les gélules, les dragées, les comprimés à noyaux multiples, les gouttes, les solutions ou suspensions buvables ; les solutés ou suspensions injectables répartis en ampoules, en flacons

multidoses ou en seringues auto-injectables ; les suppositoires ; les comprimés sublingaux.

Les compositions pharmaceutiques selon l'invention peuvent en outre renfermer un ou d'autres principes actifs d'action similaire ou complémentaire. On pourra ainsi ajouter un diurétique du type thiazidique comme le cyclothiazide ou du type triaminoptéridine comme le triamtérène ; ou un agent bêta-bloqueur comme le propranolol ou le pindola.

La posologie journalière peut varier entre des limites larges, en fonction de l'indication thérapeutique, de la voie d'administration, de l'âge du malade et de l'ancienneté de la maladie hypertensive. En règle générale, chez l'adulte la posologie s'échelonne entre 0,1 et 50 mg par prise et entre 0,1 et 150 mg par jour.

D'une manière préférée, les compositions pharmaceutiques selon l'invention renferment entre 0,1 et 25 mg, par prise unitaire, de principe actif.

L'invention comprend aussi un procédé pour préparer les composés de formule générale I caractérisé en ce que l'on fait réagir une 4-amino-pipéridine de formule générale II

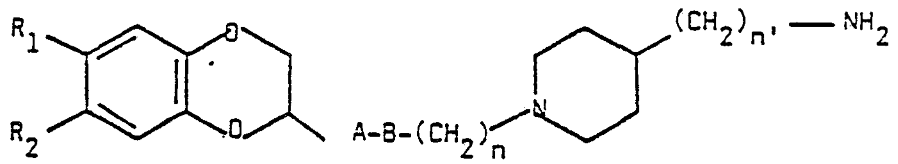

(II)

dans laquelle les substituants $R_1$, $R_2$, A, B, n et n' ont les significations fournies antérieurement, avec un réactif de cyanoimination choisi dans le groupe constitué parmi les cyanoimino isodithiocarbonates d'alcoyle de formule générale III

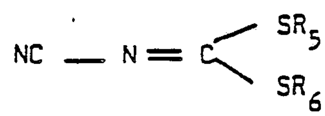

(III)

dans laquelle $R_5$ et $R_6$ sont des radicaux alcoyle inférieur et les cyanoimino isothiocarbonates mixtes d'alcoyle de formule générale IV

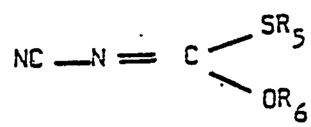

(IV)

dans laquelle $R_5$ et $R_6$ sont définis comme précédemment pour former l'isothiourée ou l'isourée de formule générale V

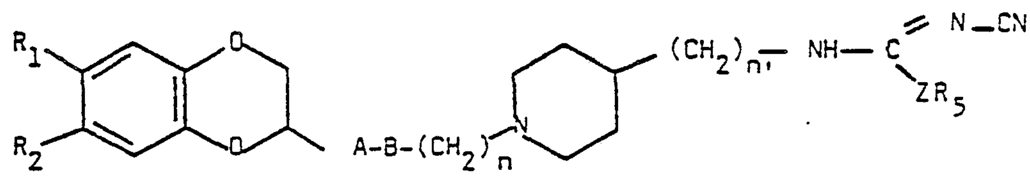

(V)

dans laquelle les substituants $R_1$, $R_2$, $R_5$, n et n' sont définis comme précédemment et Z est de l'oxygène ou du soufre, que l'on fait réagir avec une amine primaire ou secondaire de formule $R_3$ NH $R_4$ pour obtenir la cyanoguanidine de formule générale I

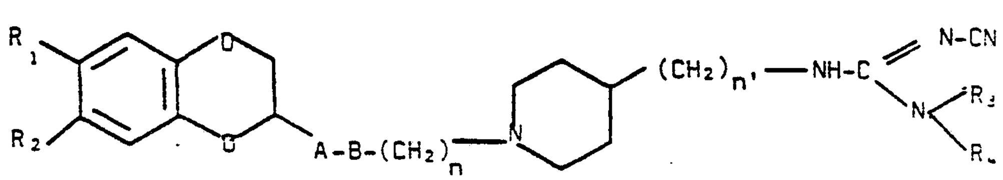

(I)

dans laquelle $R_1$, $R_2$, $R_3$, n et n' sont définis comme précédemment et $R_4$ est de l'hydrogène ou un radical alcoyle inférieur que l'on peut si désiré, lorsque $R_4$ est de l'hydrogène, acyler par action d'un dérivé fonctionnel d'acide carboxylique pour former une N'-acyl N-cyano-guanidine de formule générale I

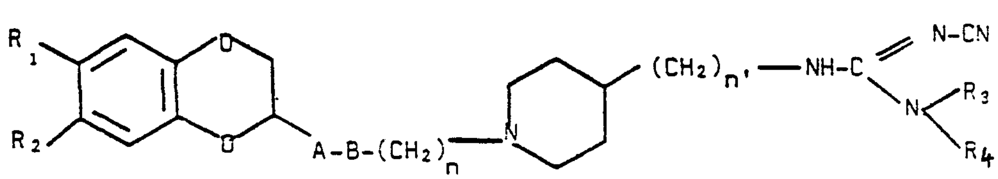

(I)

dans laquelle $R_1$, $R_2$, $R_3$, n et n' ont les définitions antérieures et $R_4$ est le reste acyle d'un acide carboxylique ayant de 1 à 12 atomes de carbone ou bien encore dédoubler par action d'un acide organique optiquement actif en ses isomères optiques ou bien encore salifier par addition d'un acide minéral ou organique.

Les 4-amino pipéridines de formule générale II sont obtenues — lorsque n' est égal à zéro — par un procédé qui consiste à condenser un ester réactif de (benzodioxanyl) — alcoyle ou hydroxyalcoyle ou cétoalcoyle avec une pipéridone bloquée, libérer la fonction cétone bloquée par hydrolyse pour former une 1-(benzodioxanyl) alcoyl pipéridine 4-one, condenser celle-ci avec l'hydroxylamine ou un de ses sels pour former l'oxime correspondante que l'on réduit au moyen d'un hydrure mixte en l'amine de formule II.

L'ester réactif de benzodioxanyl alcoyle est de préférence un chlorure, un bromure, un iodure, un méthane sulfonate ou un p. toluène sulfonate.

La réaction de condensation avec la pipéridone s'effectue en milieu polaire, de préférence en présence d'un iodure de métal alcalin. Le solvant polaire est en général la pyridine, le diméthyl formamide, la méthyl éthyl cétone ou l'hexaméthyl phosphorotriamide.

L'hydrure mixte de métal alcalin est un aluminohydrure de sodium ou de lithium, le borohydrure de sodium ou de potassium, le triméthoxy borohydrure de lithium ou le cyano-borohydrure de lithium.

L'hydrolyse de la pipéridone bloquée est effectuée soit par action d'un acide minéral ou organique aqueux ou par échange de fonction avec un acide cétonique ou aldéhyde. Le blocage peut être assuré sous forme de cétal dialcoylique ou de dioxolane substitué ou non, ou bien encore de thiocétal. Parmi les agents d'hydrolyse on citera plus particulièrement l'acide chlorhydrique, l'acide sulfurique ou l'acide p. toluène sulfonique.

Dans la formule générale II lorsque A ou B représente un groupe CHOH ou CO les composés sont obtenus au départ d'un ester de benzodioxanyl alcoyle de formule générale III

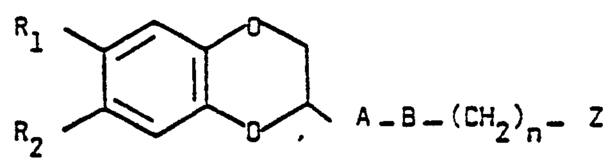

(III)

dans laquelle A ou B représente un groupe CHOH ou CO Z est un groupe acyle aisément clivable et n est égal à 0 ou 1 que l'on condense avec une pipéridone bloquée de formule générale VI

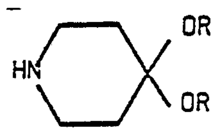

(VI)

dans laquelle R et R' sont chacun un radical alcoyle inférieur ou forment ensemble une chaîne alcoylène ayant de 2 à 4 atomes de carbone ou avec une pipéridine 4-carboxamide de formule générale VII

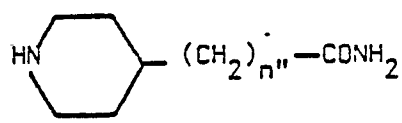

(VII)

dans laquelle n" est égal à 0 ou 1.

La (benzodioxanyl alcoyl) pipéridine résultante de formule générale VIII

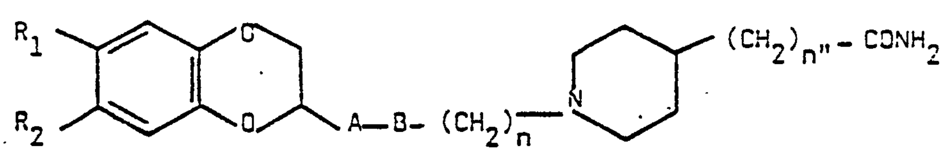

(VIII)

dans laquelle $R_1$ et $R_2$ ont les significations fournies antérieurement

n représente 0 ou 1

n" représente 0 ou 1

est ensuite réduite par action d'un hydrure mixte de métal alcalin en dérivé aminé de formule générale II

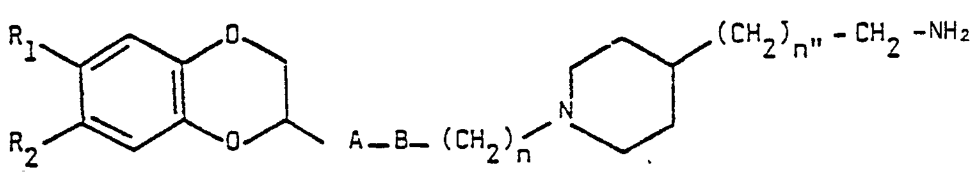

(II)

dans laquelle la définition des substituants demeure inchangée que l'on peut oxyder lorsque A ou B représentent un groupe hydroxyalcoyle en dérivé carbonylé par action d'un dérivé carbonylé en présence d'isopropylate d'aluminium.

L'invention comprend encore en tant que moyens nécessaires pour la réalisation de l'invention, les isothio- ou les iso-urées de formule générale V

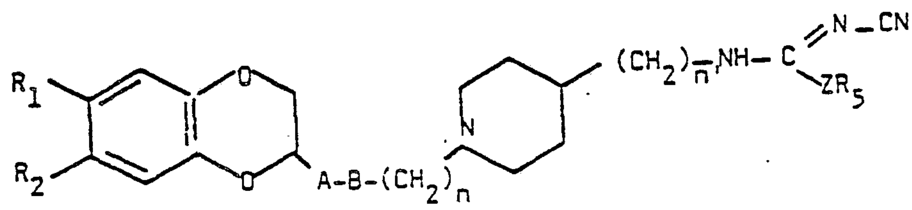

(V)

dans laquelle la définition des substituants A, B, $R_1$, $R_2$, $R_5$, n et n' demeure celle fournie précédemment et Z est de l'oxygène ou du soufre.

Les exemples suivants illustrent l'invention. Ils ne la limitent en aucune façon.

Exemple 1

4-[(2,3-dihydro [4H] 1,4-benzodioxin-2 yl) méthyl] 1-(N-cyano N'-méthylguanidinyl) pipéridine

Stade A

(2,3-dihydro [4H] 1,4-benzodioxin-2 yl méthyl)-1 pipéridone éthylènecétal

On porte 17 heures à l'ébullition avec agitation, un mélange de 184 g de 8-aza 1,4 dioxaspiro (4-5) décane, 320 g de 2-(méthylsulfonyl oxyméthyl) 1,4-benzodioxanne,180 g de carbonate de potassium et 1 500 ml de toluène.

Le milieu réactionnel est ensuite lavé à l'eau puis extrait avec une solution d'acide chlorhydrique normale.

La solution chlorhydrique est ensuite rendue basique par addition de soude diluée et extraite à l'éther.

Après séchage et concentration, on obtient 328 g du produit cherché sous forme d'huile épaisse. Elle est suffisamment pure pour être utilisée telle quelle dans le stade suivant.

Stade B

.1-[2,3-dihydro [4H] (1,4-benzodioxin-2 yl) méthyl] pipéridone-4

On porte 2 heures à l'ébullition, un mélange de 328 grammes de 1-[2,3-dihydro [4H] (1,4-benzodioxin-2 yl) méthyl] pipéridone éthylènecétal obtenu au stade A, de 550 g d'acide chlorhydrique concentré et 3 500 ml d'eau pendant 2 heures.

Après refroidissement, la solution est extraite à l'éther puis rendue basique par addition d'une solution de soude concentrée.

Après extraction à l'éther, lavage à l'eau, séchage sur sulfate de sodium et concentration, on obtient un solide qui est recristallisé dans le cyclohexane. On obtient ainsi 246 g de cristaux blancs de PFi : 95-6°.

Stade C

Oxime de la 1-[(2,3-dihydro [4H] 1,4-benzodioxin-2 yl) méthyl] pipéridone-4

On porte 1/2 heure à l'ébullition un mélange de 72 g de 1-[(2,3-dihydro [4H] 1,4-benzodioxin-2 yl) méthyl] pipéridone-4, 21 g de chlorhydrate d'hydroxylamine préalablement mis en solution dans 80 ml d'eau et 500 ml d'éthanol.

Après refroidissement, il y a cristallisation du chlorhydrate de l'oxime cherché (Fi = 250°). La base est libérée de cette dernière par addition de soude puis extraction au chloroforme.

Après séchage et concentration à sec, on obtient 65 g de l'oxime cherchée. Fi = 115°.

Stade D

1-[2,3-dihydro [4H] (1,4-benzodioxin 2-yl) méthyl] 4-aminopipéridine

On met en suspension 10 grammes d'hydrure double de lithium et d'aluminium dans 150 ml de

tétrahydrofuranne anhydre, et sous agitation à la température ordinaire on coule goutte à goutte une solution de 50 grammes d'oxime de 1-2,3 dihydro 1H (1,4 benzodioxin-2 yl) méthyl pipéridone-4 dans 300 parties de tétrahydrofuranne anhydre.

On chauffe ensuite 16 heures à 80° puis refroidit dans la glace et hydrolyse l'excès d'hydrure par addition d'eau.

Après filtration du précipité d'alumine sur célite et concentration à sec, l'huile résultante est redissoute dans l'éther. La solution éthérée est séchée sur sulfate de sodium puis la solution est concentrée à sec.

On obtient ainsi 35 grammes de l'amine cherchée sous forme d'huile qui peut être utilisée telle quelle pour la suite de la synthèse.

Stade E

1-[(2,3-dihydro [4H] 1,4-benzodioxin-2 yl) méthyl] 4-(N-cyano S-méthylisothiouréido] pipéridine

On porte 2 heures à l'ébullition 7,2 grammes de 1-[(2,3-dihydro [4H] 1,4-benzodioxin-2 yl) méthyl] 4-amino pipéridine avec 4,2 grammes de cyanoiminodithiocarbomate de diméthyle et 100 ml d'éthanol.

Au refroidissement, il y a cristallisation d'un produit qui est filtré et lavé à l'éthanol.

On obtient ainsi 6,1 grammes du produit cherché qui est purifié pour l'analyse puis recristallisé dans le méthanol. Fi = 168°.

Le cyanoiminodithiocarbonate de diméthyle est préparé selon le procédé décrit par R.J. TIMMONS et L.S. WITTENBROOK — J. org. chem. vol. 32 p. 1566 (1966).

On peut utiliser dans les mêmes conditions le cyanoiminothiocarbonate mixte de diméthyle obtenu selon le procédé décrit dans le brevet français 2.445.322. On obtient ainsi la 1-[(2,3-dihydro [4H] (1,4-benzodioxin-2 yl) méthyl] 4-N-cyano O-méthyl-isoureido pipéridine.

Stade F

1-[(2,3-dihydro [4H] 1,4-benzodioxin-2 yl) méthyl] 4-(N-cyano N-méthylquanidino) pipéridine

On met en solution 10 grammes de 1-[(2,3-dihydro [4H] 1,4-benzodioxin-2 yl) méthyl] 4-(N-cyano S-méthyl-ísothiouréido) pipéridine dans 200 ml de méthanol et en maintenant la température à 25° on fait barbotter la méthylamine gazeuse jusqu'à ce qu'il y ait une absorption de 40 grammes.

On peut suivre l'avancement de la réaction par chromatographie en couche mince dans le système toluène : 95 isopropylamine : 5.

Après environ 3 heures, la réaction est terminée et on concentre à sec ce qui donne une huile qui cristallise par traitement à l'eau.

Les cristaux obtenus sont purifiés par recristallisation dans l'isopropanol.

On obtient ainsi 8 grammes de la cyanoguanidine cherchée qui cristallise avec 1/2 molécule d'eau. Fi = 140-142°.

Exemple II

1-[(2,3-dihydro [4H] 1,4-benzodioxin 2-yl) méthyl] 4-[(N-cyano N'-méthyl) guanidinyl méthyl] pipéridine

Stade A

1-[(2,3-dihydro [4H] 1,4-benzodioxin-2 yl) méthyl] pipéridine 4-carboxamide.

On porte 3 heures à l'ébullition avec une bonne agitation, un mélange de 245 grammes de 2-tosyloxyméthyl benzodioxanne, 130 grammes de pipéridine-4-carboxamide, 140 grammes de carbonate de potassium et de 2 000 ml de xylène.

Le mélange qui devient gélatineux est ensuite traité en phase hétérogène avec agitation avec 2 litres d'eau et le solide formé est filtré, lavé abondamment à l'eau puis à l'éther isopropylique.

On obtient ainsi 190 grammes du produit cherché sous forme de cristaux blancs, fondant à 135-6°, assez purs pour la suite de la synthèse.

Stade B

1-[2,3-dihydro [4H] (1,4-benzodioxin 2-yl) méthyl] 4-aminométhyl pipéridine.

A une suspension de 75 grammes d'hydrure de lithium et d'aluminium dans 500 ml de tétrahydrofuranne, on coule à température ambiante sous agitation, une solution de 190g de [2,3-dihydro [1H] (1,4-benzodioxin-2 yl) méthyl] pipéridine 4-carboxamide dans 1 500 ml de tétrahydrofuranne. Une fois l'addition terminée on chauffe 2 heures à l'ébullition.

L'excès d'hydrure de lithium et d'aluminium est hydrolysé par addition d'eau en refroidissant.

On filtre ensuite sur célite et la solution est concentrée à sec.

L'huile obtenue est dissoute dans l'éther et la solution obtenue séchée sur sulfate de sodium anhydre.

Après concentration à sec, on obtient 124 grammes de l'amine cherchée sous forme d'une huile épaisse. Le produit est assez pur pour pouvoir être utilisé pour la poursuite de la synthèse.

Stade C

1-[2,3-dihydro [4] (1,4-benzodioxin 2-yl) méthyl] 4-[N-cyano S-méthylisothiouréïdo) méthyl] pipéridine

On porte 4 heures à l'ébullition un mélange de 65,5 grammes 1-[(2,3-dihydro [4H] 1,4-benzodioxin-2 yl) méthyl] 4-aminométhyl pipéridine, 36,5 grammes de cyanoiminodithiocarbamate de diméthyle et 500 ml d'éthanol.

Le produit qui cristallise par refroidissement est filtré, lavé à l'éthanol et séché. On obtient 84 grammes d'iso-urée sous forme de cristaux blancs de Fi = 148°.

Stade D

1-[(2,3-dihydro [4H] 1,4-benzodioxin-2yl) méthyl] 4-[(N-cyano N'-méthyl guanidinyl) méthyl] pipéridine.

On met en suspension 10 grammes de 1-[(2,3-dihydro [4H] (1,4-benzodioxin 2-yl) méthyl] 4-[(N-cyano S-méthylisothiouréïdo) méthyl] pipéridine dans 120 ml de méthanol et on fait barbotter de la méthylamine gazeuse en maintenant la température vers 30°.

Il y a dissolution progressive et on arrête le barbottage après prise de 50 grammes de méthylamine.

On peut suivre la réaction en chromatographie en couche mince en utilisant le mélange chloroforme — Isopropylamine comme éluant.

Après 2 heures de barbottage, la réaction est terminée et on évapore à sec la solution réactionnelle. On lave l'huile obtenue avec de l'éther isopropylique.

Après séchage, on obtient un solide cassant mais non cristallisé. Fi peu nette vers 100°.

Exemple III

1-[(2,3-dihydro [4H] 1,4-benzodioxin 2-yl) méthyl] 4-[(N-cyano N'-allyl) guanidinyl méthyl] pipéridine

On porte 16 heures à l'ébullition un mélange de 6,5 grammes de 1-[2,3-dihydro [4H] (1,4-benzodioxin-2 yl) méthyl] 4-(N-cyano N'-S-méthylisothiouréïdo) méthyl] pipéridine obtenu comme au stade C de l'exemple II, 20 ml de pyridine et 30 grammes d'allylamine.

La solution est ensuite concentrée à sec et traitée par l'éther isopropylique ce qui permet d'obtenir 6,5 grammes de cristaux blancs. Fi = 136-137°, qu'il est possible de recristalliser dans l'isopropanol. Le point de fusion reste inchangé.

Exemple IV

1-[(2,3-dihydro [4H] 1,4-benzodioxin 2-yl) méthyl] 4-[(N-cyano N'-(méthyl-1) propyl) guanidinyl méthyl] pipéridine

On porte 16 heures à l'ébullition, un mélange de 3 grammes de 1-[(2,3-dihydro [4H] (1,4-benzodioxin 2-yl) méthyl] 4-(N-cyano S-méthyl isothiouréïdo) méthyl pipéridine, 10 ml de pyridine et 30 grammes de butylamine secondaire.

Aprés évaporation à sec, l'huile obtenue est traitée à l'éther isopropylique ce qui permet d'obtenir un solide sous forme de cristaux blancs qui sont recristallisés dans l'isopropanol.

On obtient 2 grammes de dérivé secbutylé de PFi : 145°.

Exemple V

1-[(2,3-dihydro [4H] 1,4-benzodioxin 2-yl) méthyl] 4-[(N-cyano N'-propyl) guanidinylméthyl] pipéridine

On porte 18 heures à l'ébullition un mélange de 4 grammes de 1-[(2,3-dihydro [4H] 1,4-benzodioxin-2 yl) méthyl 4-(N-cyano S-méthylisothiouréïdo) méthyl pipéridine, 15 ml de pyridine et 30 grammes de n-propylamine.

Après concentration à sec, l'huile obtenue est traitée par l'éther isopropylique pour donner 3,5 grammes d'un solide sous forme de cristaux qui sont purifiés par recristallisation dans l'isopropanol. Fi = 130-131°.

## Exemple VI

1-[(2,3-dihydro [1H] 1,4-benzodioxin 2-yl) méthyl] 4-[(N-cyano N'-cyclopropyl) guanidinylméthyl] pipéridine

On porte 18 heures à l'ébullition un mélange de 6 grammes de 1-[(2,3 dihydro [4H] 1,4-benzodioxin-2 yl) méthyl] 4-(N-cyano S-méthylisothiouréïdo)-méthyl pipéridine, 20 ml de pyridine et 30 grammes de cyclopropylamine.

Après concentration à sec, l'huile obtenue est traitée à l'éther isopropylique ce qui donne 5,4 grammes de cristaux blancs qui sont recristallisés dans l'éthanol. Fi = 162°.

## Exemple VII

1-[(2,3-dihydro [4H] 1,4-benzodioxin 2-yl) méthyl] 4-(N-cyano N'-triméthyl-1,2,2 propyl) guanidinylméthyl pipéridine.

On porte 16 heures à l'ébullition un mélange de 20 grammes de 1-[(2,3-dihydro [4H] 1,4-benzodioxin 2-yl) méthyl] 4-(N-cyano S-méthylisothiouréïdo) méthyl pipéridine, 80 grammes de 3,3-diméthyl 2-butylamine et 20 ml de pyridine.

On concentre à sec et on lave l'huile obtenue plusieurs fois à l'éther isopropylique et après séchage sous vide, on obtient le produit cherché sous forme d'un solide vitreux fondant progressivement vers 100°.

Bien que non cristallisé, le produit est pur et ne présente qu'une seule tache en chromatographie en couche mince avec le mélange chloroforme-isopropylamine 9-1 comme éluant.

## Exemple VIII

1-[2,3 dihydro [4H] 1,4-benzodioxin 2-yl)-méthyl] 4-(N-cyano N' allyl) guanidinyl pipéridine.

On porte 14 heures à l'ébullition, un mélange de 10 grammes de 1-[(2,3 dihydro [4H] 1,4 benzodioxin 2 yl)-méthyl] 4-(N-cyano S-isothiouréido) pipéridine et 60 ml d'allylamine.

La solution est concentrée à sec ce qui donne une huile qui, traitée par l'éther isopropylique, fournit des cristaux blancs.

Ceux-ci sont lavés à l'éther isopropylique, séchés et recristallisés dans l'isopropanol pour donner 7,5 g du dérivé allylé cherché sous forme de cristaux incolores de Fi = 140°.

## Exemple IX

1-[(2,3 dihydro [4H] 1,4-benzodioxin 2-yl) méthyl] 4-[(N-cyano N'-morpholino guanidinyl méthyl] pipéridine

On porte à l'ébullition pendant 20 heures un mélange de 5 g de 1-4-[2,3-dihydro [4H] 1,4-benzodioxin-2 yl) méthyl] 4-(N-cyano S-méthyl isothiouréïdo) méthyl] pipéridine et 50 ml de morpholine. On évapore ensuite la solution claire à sec sous pression réduite et on obtient un produit huileux qui se solidifie par trituration avec de l'éther éthylique.

Le solide obtenu est filtré, lavé à l'éther, séché et recristallisé dans l'acétonitrile après décoloration au noir animal.

On obtient ainsi 1,3 g du dérivé morpholinique cherché. Il fond à 164°.

## Exemple X

Etude pharmacologique des composés selon l'invention

a) Détermination de la toxicité aiguë

Une dose léthale 50 approchée a été déterminée par voie orale, sur des lots de 8 souris femelles ECPS d'élevage CESAL par la méthode de CAMPBELL D.E.S. et RICHTER W. (Acta Pharmacol. and Toxicol. 1967-25-345).

Les animaux ont été gardés en observation pendant 5 jours. Les doses léthales moyennes des produits selon l'invention s'échelonnent entre 110 et 880 mg/kg P.O.

b) Détermination d'un effet sur le système nerveux central

Les composés suivant l'invention ont été administrés par voie orale à des lots de 5 souris femelles EOPS d'élevage CESAL et à des doses variant de 5 à 50 mg/kg. Des tests : traction-cheminée-évasion-planche à trous-température-ptosis-électrochocs ont été effectués 1 heure après gavage.

Ces produits entraînent une légère sédation avec baisse de température, un léger ptosis et une diminution du comportement d'investigation.

c) Mise en évidence d'un effet vasodilatateur

L'effet vasodilatateur périphérique des composés selon l'invention a été observé chez le Rat, particulièrement au niveau des pattes postérieures pour lesquelles on enregistre parallèlement une augmentaion de la température cutanée de 3 à 4 °C. Cet effet apparaît, selon les produits, pour des doses variant de 1 à 5 mg/kg.

Cet effet vaso-dilatateur s'accompagne d'un effet inhibiteur très important sur la diurèse.

d) Détermination d'un effet anti-hypertensif

L'essai a été réalisé sur des Rats mâles hypertendus vigiles, obtenus après ligature de l'aorte abdominale.

Les produits selon l'invention ont été administrés par voie orale à 2,5 ou 10 mg/kg. Ils entraînent une baisse de pression artérielle nette et prolongée.

D'autre part, ces mêmes composés entraînent également une très nette hypotension pour des doses de 100 et 500 µg/kg lorsqu'ils sont administrés par voie intra-veineuse chez le Rat normotendu anesthésié.

Exemple XI

1-[(6-méthyl 2,3-dihydro [4H] 1,4-benzodioxin-2 yl) méthyl] 4-[(N-cyano N'-allyl) guanidinyl méthyl] pipéridine

Stade A

2-hydroxyméthyl 6-méthyl benzodioxanne 1,4

Dans un réacteur muni d'une agitation et d'une régulation de la température, on mélange 298 g de 4-méthylpyrocatéchol et 288 g d'épichlorhydrine puis on chauffe à 70°.

On coule alors une solution formée de 106 g d'hydroxyde de sodium dans 800 ml d'eau.

La réaction est isothermique et la température se maintient aux alentours de 80-90°.

On chauffe ensuite 2 h 30 à 70° puis verse dans l'eau.

On extrait à l'acétate d'éthyle et la phase organique séparée est lavée à la soude diluée puis à l'eau salée jusqu'à neutralité.

Après séchage et concentration, on obtient une huile qui est distillée sous vide et sous azote. On obtient le benzodioxanne souos forme d'une huile épaisse de point d'ébullition élevé (P. Eb 0,2 de 110 à 240). Poids obtenu = 188 g.

Stabe B

2-tosyloxyméthyl 6-méthyl benzodioxanne 1,4

A une solution refroidie de 188 g de 2-hydroxyméthyl 6-méthyl benzodioxanne 1,4 obtenu au stade A dans 350 ml de pyridine, on ajoute par petites portions 199 g de chlorure d'acide p. toluène sulfonique en maintenant la température à 20-25°.

On agite ensuite 18 heures à la température ambiante.

Le mélange est ensuite versé dans l'eau et extrait au chloroforme. Les phases chloroformiques sont séparées. Après lavage à l'acide chlorhydrique dilué puis à l'eau salée, séchage et concentration, on obtient une huile orangée.

Cette huile est dissoute à chaud dans 1 litre d'éther isopropylique, il y a cristallisation au refroidissement.

On obtient ainsi après filtration et séchage 204 g du tosylate cherché sous forme de cristaux fondant à 84-86°.

Stade C

1[(6-méthyl 1,4 benzodioxanne 2-yl) méthyl)] pipéridine-4 carboxamide

On chauffe 7 heures au reflux avec agitation un mélange de 167 g du tosylate préparé au stade B, 64 g de pipéridine 4-carboxamide, 69 g de carbonate de potassium dans 1 litre de xylène.

Après refroidissement, on traite à l'eau et filtre.

Le solide est lavé abondamment à l'eau puis à l'éther isopropylique et séché.

On obtient 82 g de pipéridine carboxamide cherché sous forme d'un solide blanc fondant à 138°.

Stade D

1[(6-méthyl 1,4 benzodioxan 2-yl) méthyl] 4-aminométhyl pipéridine

Dans une suspension agitée de 30 g d'hydrure de lithium et d'aluminium dans 750 ml de tétrahydrofuran, on introduit lentement une suspension de 88 g de pipéridine carboxamide précédemment préparé au stade C en solution dans 400 ml de tétrahydrofuran à la température ordinaire.

On chauffe ensuite 3 heures à l'ébullition. L'excès d'hydrure de lithium et d'aluminium est hydrolysé par addition lente d'eau en refroidissant.

Après filtration sur célite, la solution est concentrée à sec ce qui donne l'amine cherchée sous forme d'huile, pure en chromatographie en phase vapeur. Poids obtenu = 59 g.

Stade E

1[(6-méthyl 2,3 dihydro [4H] 1,4-benzodioxan 2-yl) méthyl] 4-(N-cyano S-méthylisothiouréïdo méthyl) pipéridine

On chaufe 4 heures à l'ébullition un mélange de 58,4 g de 1-[(6-méthyl 2,3-dihydro [4H] 1,4 benzodioxin-2 yl) méthyl] 4-aminométhyl pipéridine, de 30,9 g de cyanoimino dithiocarbonate de diméthyle dans 400 ml d'éthanol.

Après refroidissement et une nuit de repos, le produit cristallise.

Les cristaux sont filtrés, lavés à l'éthanol et séchés ce qui donne 54 g du produit cherché sous forme de cristaux blancs fondant à Fi = 145°.

Stade F

1-[(6-méthyl 2,3-dihydro [4H] 1,4-benzodioxin 2-yl) méthyl] 4-[(N-cyano N'-allyl) guanidinyl méthyl] pipéridine.

On chauffe 16 heures à l'ébullition un mélange de 15 g de 1-[(6-méthyl 2,3-dihydro [4] 1,4-benzodioxin 2-yl) méthyl] 4-(N-cyano S-méthylisothiouréïdométhyl) pipéridine, de 30 ml de pyridine et de 70 ml d'allylamine.

Par concentration à sec on obtient une huile qui est triturée avec de l'éther isopropylique jusqu'à cristallisation.

Le solide ainsi obtenu est purifié par recristallisation dans l'isopropanol ce qui permet d'obtenir 11 g du produit cherché sous forme de cristaux fondant à PFi = 118°.

Exemple XII

1-[(6-méthyl 2,3-dihydro [4] 1,4-benzodioxin 2-yl) méthyl] 4-(N-cyano N'-méthyl) guanidinyl méthyl pipéridine

Dans une suspension agitée de 15 g de 1-[(6-méthyl 2,3-dihydro [4H] 1,4-benzidioxin 2-yl) méthyl] 4-(N-cyano S-méthylisothiouréïdo méthyl) pipéridine dans 200 ml de méthanol, on fait barbotter en maintenant la température vers 25°, de la méthylamine gazeuse.

Il y a dissolution progressive et lorsque la solution est totale, la quantité de méthylamine absorbée est de 80 g.

On agite alors 18 heures à la température ambiante.

La solution est ensuite concentrée à sec ce qui donne une huile qui cristallise par trituration avec de l'éther.

Le produit obtenu est purifié ensuite par recristallisation dans l'isopropanol.

On obtient 9,8 g de cristaux incolores fondant à PFi = 130°.

Exemple XIII

1-[(2,3-dihydro [4H] 1,4-benzodioxin-2 yl éthyl] 4-[(N-cyano N'-allyl) guanidinylméthyl] pipéridine

Stade A

3,4-dibromobutyronitrile

Dans un réacteur muni d'une bonne agitation, on place 356 ml soit 295 g de cyanure d'allyle (d = 0,83) et 1,4 litre d'éther de pétrole de point d'Eb 30-40°.

La solution est refroidie à —15° et on ajoute goutte à goutte en maintenant à cette température,

228 ml de brome soit 704 g (d = 3,12). Le 3,4 dibromobutyronitrile formé se sépare et par décantation on en obtient 480 ml qui est utilisé tel quel immédiatement.

Stade B

2-cyanométhyl benzodioxanne 1,4

Dans un réacteur muni d'une bonne agitation, on place 440 g de pyrocatéchol en solution dans 3,2 litres d'acétone anhydre et 114 g de carbonate de potassium. On porte le tout au reflux du solvant. On coule alors 30 ml du dérivé dibromé préparé au stade A et une fois l'introduction terminée on rajoute 114 g de carbonate de potassium puis coule encore une fois 30 ml de dérivé dibromé préparé au stade A. On répète 16 fois cette opération ce qui revient à introduire 480 ml de 3,4-dibromobutyronitrile et 1 824 g de carbonate de potassium.

On chauffe ensuite 20 heures à l'ébullition.

Après refroidissement, le bromure de potassium formé est filtré et lavé à l'acétone et la solution acétonique est concentrée sous pression réduite.

Le résidu sec est traité par l'eau et extrait à l'éther ; la phase éthérée est lavée avec de la soude diluée puis à l'eau salée jusqu'à neutralité.

Après séchage et concentration à sec on obtient une huile jaune foncée.

Cette huile est distillée sous vide ce qui permet d'obtenir 557 g d'une huile claire qui cristallise (Eb 0,1 = 120-123°).

On peut recristalliser le benzodioxane par dissolution à chaud du produit dans 250 ml d'isopropanol à chaud et en laissant cristalliser sous agitation.

On a ainsi une prise en masse et les cristaux formés sont broyés, essorés et lavés à l'éther de pétrole (eb 30-40°). On obtient ainsi 538 g de produit pur fondant à PFi = 66-67°.

Stade C

Acide (1,4 benzodioxanne-2 yl) acétique.

On chauffe 48 heures à l'ébullition un mélange de 175 g de 2-cyanométhyl [benzodioxanne 1,4] 250 ml d'eau, 250 ml d'acide acétique et 100 ml d'acide sulfurique concentré.

On verse ensuite le mélange dans 5 litres d'eau et agite.

Les cristaux obtenus sont filtrés, lavés à l'eau et séchés ce qui donne 172 g d'acide (benzodioxanne-2 yl) acétique fondant à PFi = 95°.

Stade D

2-(hydroxy-1 éthyl) (1,4 benzodioxanne)

Dans un réacteur muni d'une agitation et d'un réfrigérant, on place une suspension de 50 g d'aluminohydrure de lithium dans 1,5 l de tétrahydrofuran.

On introduit ensuite goutte à goutte une solution de 172 g de l'acide préparé au stade C en solution dans 0,50 l de tétrahydrofuran.

Une fois l'introduction terminée, on chauffe 18 h au reflux du solvant. L'excès de réactif hydrogénant est alors détruit par addition d'eau à froid goutte à goutte puis on filtre le précipité d'alumine sur célite.

La solution est alors concentrée à sec, reprise par 1 litre d'éthanol, filtrée, puis concentrée à sec à nouveau.

L'huile obtenue est distillée sous pression réduite ce qui permet d'obtenir 132 g de l'alcool cherché sous forme d'une huile épaisse incolore.

Eb 0,05 = 119-122°.

Stade E

Tosylate de 2-(hydroxy-1 éthyl) (1,4 benzodioxanne)

A une solution refroidie à 5-10° de 132 g de l'alcool précédemment préparé dans 260 ml de pyridine, on ajoute petit à petit en maintenant à cette température, 139,4 de chlorure de p-toluènesulfonyle.

Après agitation 4 heures à la température ambiante, on verse dans 4 litres d'eau et épuise au chlorure de méthylène.

La solution méthylénique est lavée à l'acide chlorhydrique puis à l'eau salée.

Après séchage de la phase organique et concentration, on obtient une huile jaune qui est dissoute à chaud dans 800 ml d'éthanol.

Il y a cristallisation au refroidissement.

Les cristaux sont filtrés, lavés et séchés. On obtient 75 g du tosylate cherché fondant à Fi = 95°.

Stade F

1-[(1,4 benzodioxanne 2-yl) éthyl] pipéridine-4 carboxamide

On chauffe 7 heures à l'ébullition sous agitation un mélange de 104 g du tosylate préparé au stade E, de 40 g de pipéridine-4 carboxamide et de 43 g de carbonate de potassium dans 750 ml de xylène.

Après refroidissement le mélange est repris à l'eau en agitant et filtré.

Le solide est lavé abondamment à l'eau puis à l'éther isopropylique.

Après séchage on obtient 78 g du produit cherché de PFi = 190° pouvant être utilisé tel quel pour la suite de la synthèse.

Stage G

1-[(1,4 benzodioxanne 2-yl) éthyl] 4-aminométhyl pipéridine

On fait une suspension de 30 g d'hydrure de lithium et d'aluminium dans 1 litre de tétrahydrofuran anhydre et dans cette suspension on introduit par petites portions 78 g de l'amide précédemment préparé au stade F sous forme solide. La température se maintient à environ 35-45°.

Un fois l'addition terminée le mélange est porté 3 heures au reflux.

L'excès d'aluminohydrure de lithium est détruit par addition d'eau lentement en refroidissant.

Après filtration sur célite, la solution est concentrée à sec ce qui donne une huile qui est reprise par l'éther.

Après séchage sur sulfate de sodium et concentration à sec sous vide on obtient l'amine cherchée sous forme d'une huile jaune pure en chromatographie en phase vapeur. Poids obtenu : 73 g.

Stade H

1-[(2,3-dihydro [4H] 1,4-benzodioxin 2-yl) éthyl] 4-(N-cyano S-méthylisothiouréïdo)-méthyl pipéridine.

On chauffe 4 heures à l'ébullition un mélange de 73 g de 1-[(2,3-dihydro [4H] 1,4-benzodioxin 2-yl) éthyl] 4-aminométhylpipéridine et 38,6 g de cyanoiminodithiocarbonate de diméthyle dans 500 ml d'éthanol.

Il y a cristallisation au refroidissement.

Après filtration, lavage à l'éthanol des cristaux et séchage sous vide, on obtient 85 g du produit cherché, sous forme de cristaux blancs présentant un double point de fusion d'abord à 145° puis solidification et fusion à 155°.

Stade I

1-[(2,3 dihydro [4] 1,4 benzodioxin 2-yl) éthyl] 4-[(N-cyano N'-allyl) guanidinylméthyl] pipéridine

On porte 16 heures à l'ébullition un mélange de 15 g de 1-[(2,3-dihydro [4H] 1,4-benzodioxin 2-yl) éthyl] 4-(N-cyano S-méthylisothiouréïdo)-méthyl pipéridine, de 50 ml de pyridine et 50 ml d'allylamine.

Après concentration à sec, on obtient une pâte qui cristallise par trituration avec de l'éther isopropylique. Les cristaux obtenus sont filtrés, lavés et séchés sous vide.

Par recristallisation dans l'isopropanol, on obtient 14 g de produit pur PFi = 131°.

## Exemple XIV

1-[(2,3-dihydro [4H] 1,4-benzodioxin 2-yl) éthyl] 4-[(N-cyano N'-méthyl) guanidinylméthyl] pipéridine

Dans une suspension agitée de 15 g de 1-[(2,3-dihydro [4H] 1,4-benzodioxin 2-yl) éthyl] 4-(N-cyano S-méthylisothiouréïdo)-méthyl pipéridine obtenue à l'exemple XII dans 250 ml de méthanol on fait barbotter de la méthylamine gazeuse en maintenant la température vers 25-30°.

Il y a dissolution progressive et celle-ci est totale lorsque l'on a absorbé 120 g de méthylamine.

On agite ensuite 18 heures à la température ambiante puis concentre à sec.

On obtient une pâte qui cristallise par trituration avec de l'éther.

On purifie ensuite par recristallisation dans l'isopropanol ce qui permet d'obtenir 11 g de produit sous la forme de cristaux blancs fondant à PFi = 125°.

## Exemple XV

1-[(2,3-dihydro [4H] 1,4-benzodioxin-2 yl) (2-hydroxyéthyl)] 4-[(N-cyano N'-allyl) guanidinylméthyl] pipéridine

Stade A

1-[(2,3-dihydro [4H] 1,4-benzodioxin-2 yl) (hydroxy-2 éthyl)] pipéridine 4-carboxamide

On porte 7 heures à reflux sous agitation dans un réacteur muni d'un séparateur du type « Dean stark », 81 g de 2-[(1-bromo 2-hydroxy) éthyl] benzodioxanne 1,4 obtenu selon le procédé décrit dans J. Med. Chem. 13 (1970) 175 40 g de pipéridine-4 carboxamide, 40 g de carbonate de potassium et 750 ml de xylène.

Après refroidissement, le solide est filtré, lavé abondamment à l'eau, puis à l'isopropanol. On obtient 66 g de cristaux de couleur crème fondant à PFi = 186°.

Le produit est assez pur pour être utilisé tel quel pour la suite de la synthèse.

Stade B

1-[1,4 benzodioxanne 2-yl (hydroxy-2 éthyl)] 4-aminométhylpipéridine.

A température ordinaire et sous agitation, on prépare une suspension de 30 g d'aluminohydrure de lithium dans 1 litre de tétrahydrofuranne.

On ajoute ensuite par petites portions 66 g de 1-[2,3-dihydro [4H] (1,4 benzodioxin-2 yl) (2-hydroxy éthyl)] pipéridine 4-carboxamide broyé.

Une fois l'introduction terminée on chauffe 3 heures à l'ébullition.

On hydrolyse ensuite l'excès d'aluminohydrure de lithium par addition d'eau en maintenant la température à 0°.

Après filtration sur célite, la solution résultante est concentrée à sec sous vide ce qui fournit 57 g de l'amine cherchée sous forme d'une huile orangée claire.

Stade C

1-[1,4-benzodioxanne-2 yl (hydroxy-2 éthyl) 4-(N-cyano S-méthylisothiouréïdométhyl) pipéridine

On chauffe 4 heures à l'ébullition un mélange de 58 g de 1-[1,4 benzodioxanne-2 yl (2-hydroxy éthyl)] 4-aminométhyl pipéridine et de 29 g de N-cyanoiminodithiocarbonate de diméthyle dans 400 ml d'éthanol.

La solution est ensuite concentrée à sec et reprise par l'éther isopropylique, ce qui permet d'obtenir des cristaux qui sont filtrés, lavés à l'éther isopropylique et séchés.

Par recristallisation dans l'isopropanol on obtient 58 g de la pipéridine cherchée fondant à Fi = 128°.

Stade D

1-[(2,3-dihydro [4H] 1,4-benzodioxin 2-yl) (2-hydroxy éthyl)] 4-[(N-cyano N'-allyl) guanidinyl méthyl] pipéridine

On porte 16 heures à l'ébullition un mélange de 15,6 g de 1-[(2,3-dihydro [4H] 1,4-benzodioxin-2 yl) (2-hydroxyéthyl)] 4-[(N-cyano S-méthylisothiouréïdo)-méthyl] pipéridine, 50 ml de pyridine et 70 ml d'allylamine.

Après concentration à sec on obtient une huile épaisse qui est cristallisée par trituration avec de l'éther éthylique.

On obtient ainsi 17,4 g de cristaux qui sont purifiés par recristallisation dans l'isopropanol par chaud et froid.

La 1-[(2,3-dihydro [4H] 1,4-benzodioxin-2 yl) (2-hydroxyéthyl)] 4-[(N-cyano N'-allyl) guanidinyl méthyl] pipéridine se présente sous forme de cristaux incolores fondant à 30°.

Exemple XVI

1-[(2,3-dihydro [4] 1,4-benzodioxin 2-yl) (2-hydroxy éthyl)] 4-[(N-cyano N'-méthyl) guanidinyl méthyl] pipéridine

Dans une suspension agitée de 15,6 g de 1-[(2,3-dihydro [4H] 1,4-benzodioxin-2 yl) (hydroxy-2 éthyl] 4[(N-cyano S-métylisothiouréïdo)-méthyl] pipéridine obtenue selon le procédé de l'exemple XV dans 250 ml de méthanol, on fait barbotter de la méthylamine gazeuse en maintenant la température aux alentours de 25-30°.

On arrête le barbottage lorsque la dissolution est totale et à ce moment la quantité de méthylamine absorbée est de 80 g.

On agite ensuite 18 heures à température ambiante.

La solution est alors évaporée à sec ce qui donne une huile épaisse qui est reprise à l'acétonitrile. Après quelques heures il y a formation de cristaux qui sont séparés par filtration, lavés et séchés. On obtient ainsi 14,6 g du produit cherché qui peut être recristallisé dans l'isopropanol. Après recristallisation il fond à Fi = 150°.

**Revendications**

1. Les pipéridinyl-4 (N-cyano N'-R) guanidines choisies dans le groupe constitué par les pipéridines de formule générale I

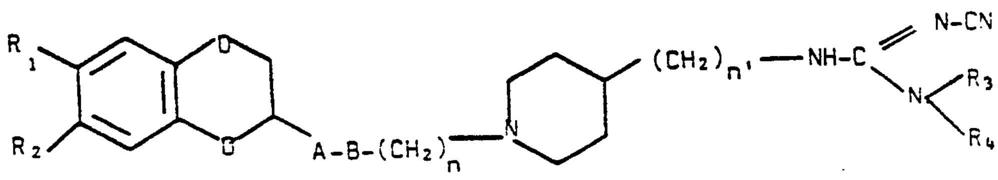

(I)

dans laquelle

A représente $—CH_2—$ ou une liaison simple et B représente —CHOH— ou —C = O ou bien A représente —CHOH— ou —C = O et B représente une liaison simple ou $—CH_2—$

n est égal à 0 ou 1

les pipéridines de formule générale $I_A$

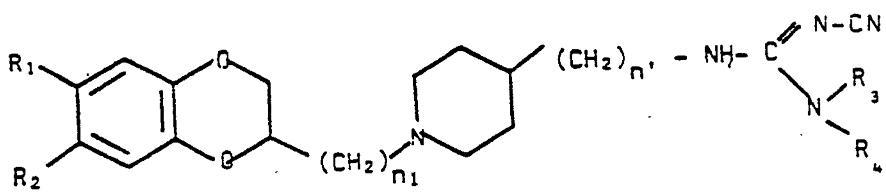

($I_A$)

dans laquelle $n_1$ est un nombre entier variant de 1 à 3

$R_1$ et $R_2$ identiques ou différents, représentent de l'hydrogène, un radical alcoyle inférieur ayant de 1 à 6 atomes de carbone, un radical alcoxy inférieur ayant de 1 à 6 atomes de carbone, un halogène ou un radical trifluorométhyle.

$R_3$ représente un radical alcoyle inférieur ayant de 1 à 6 atomes de carbone, un radical alcényle inférieur ayant de 2 à 4 atomes de carbone, un radical cycloalcoyle inférieur ayant de 3 à 7 atomes de carbone ou un radical hétérocyclanyle ayant de 5 à 7 chaînons.

$R_4$ représente de l'hydrogène, un radical alcoyle inférieur ayant de 1 à 6 atomes de carbone ou le reste acyle d'un acide organique carboxylique ayant de 1 à 12 atomes de carbone.

ou bien $R_3$ et $R_4$ forment ensemble le reste alcoylène d'un hétérocycle azoté comportant éventuellement un autre hétéroatome, ayant de 5 à 7 maillons

n' est égal à 0 ou 1 et

les sels des composés de formule générale I et $I_A$ avec un acide minéral ou organique.

2. Les sels d'addition avec un acide minéral ou organique — de préférence un acide thérapeutiquement compatible — d'un composé de formule générale I selon la revendication 1.

3. Les formes optiquement -actives des composés de formule générale I ainsi que les diastéréoisomères des composés de formule générale I selon la revendication 1.

4. Les pipéridines de formule générale $I_A$ selon la revendication 1 choisies dans le groupe constitué par

les pipéridines de formule générale

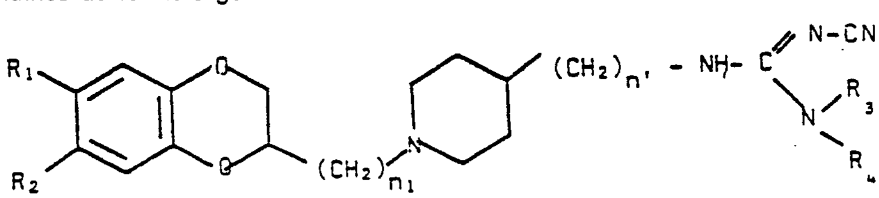

($I_A$)

dans laquelle les substituants $R_1$, $R_2$, $R_3$, $R_4$ et n' sont définis comme précédemment et

les sels d'addition des composés de formule $I_A$ avec un acide minéral ou organique.

5. Les pipéridines selon la revendication 1 de formule générale $I_B$ choisies dans le groupe constitué par

les pipéridines de formule générale

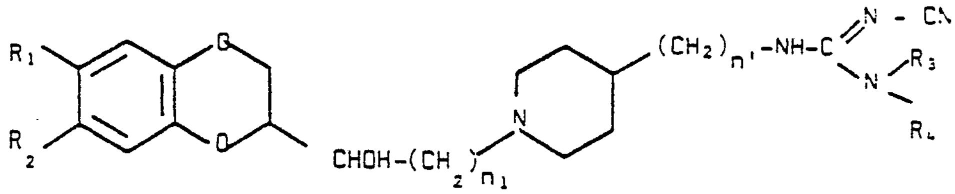

($I_B$)

dans laquelle les substituants $R_1$, $R_2$, $R_3$, $R_4$ et n' sont définis comme précédemment et $n^1$ est un nombre entier variant de 0 à 2 et

les sels d'addition des composés de formule générale $I_B$ avec un acide minéral ou organique.

6. Les pipéridines selon la revendication 1 de formule générale $I_C$ choisies dans le groupe constitué par

les pipéridines de formule générale

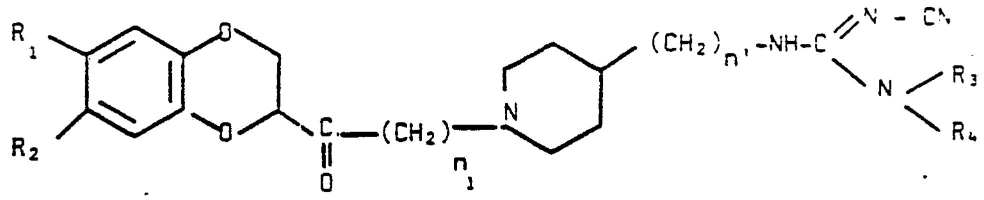

($I_C$)

dans laquelle les substituants $R_1$, $R_2$, $R_3$, $R_4$ et n' sont définis comme précédemment et $n_1$ est un nombre entier variant de 0 à 2 et

les sels d'addition des composés de formule générale $I_C$ avec un acide minéral ou organique.

7. La 1-[(2,3-dihydro [4H] 1,4-benzodioxin-2 yl) méthyl] 4-(N-cyano N'-méthyl) guanidinyl] pipéridine selon la revendication 1.

8. La 1-[(2,3-dihydro [4H] 1,4-benzodioxin-2 yl) méthyl] 4[(N-cyano N'-méthyl) guanidinyl méthyl] pipéridine selon la revendication 1.

9. La 1-[(2,3-dihydro [4H] 1,4-benzodioxin-2 yl) méthyl] 4-[(N-cyano N'-allyl) guanidinyl méthyl] pipéridine selon la revendication 1.

10. Les compositions pharmaceutiques renfermant à titre de principe actif au moins un composé selon l'une des revendications 1 à 9 en association ou en mélange avec un excipient ou un véhicule inerte, non toxique, pharmaceutiquement acceptable.

11. Un procédé pour préparer les composés de formule générale I selon la revendication 1 caractérisé en ce que l'on fait réagir une 4-amino-pipéridine de formule générale II

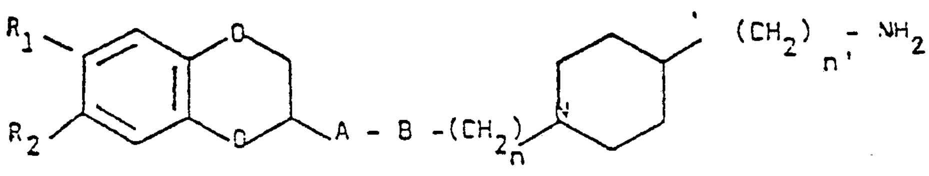

(II)

ou de formule générale $II_A$

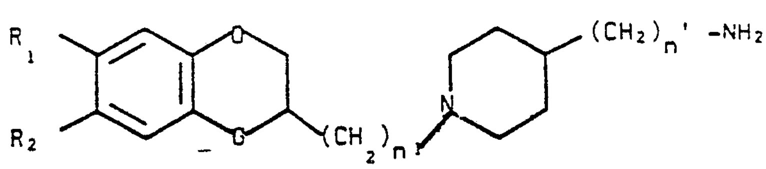

($II_A$)

dans lesquelles $R_1$, $R_2$, A, B, $n_1$ et n' sont définis comme ci-dessus avec un réactif de cyano-imination choisi dans le groupe constitué parmi les cyanoimino isodithiocarbonates d'alcoyle de formule générale III

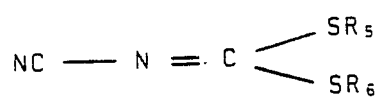

(III)

dans laquelle $R_5$ et $R_6$ sont des radicaux alcoyle inférieur et les cyanoimino isothiocarbonates mixtes d'alcoyle de formule générale IV

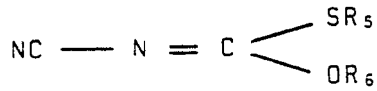 (IV)

dans laquelle $R_5$ et $R_6$ sont définis comme précédemment pour former l'isothiourée ou l'isourée de formule générale V

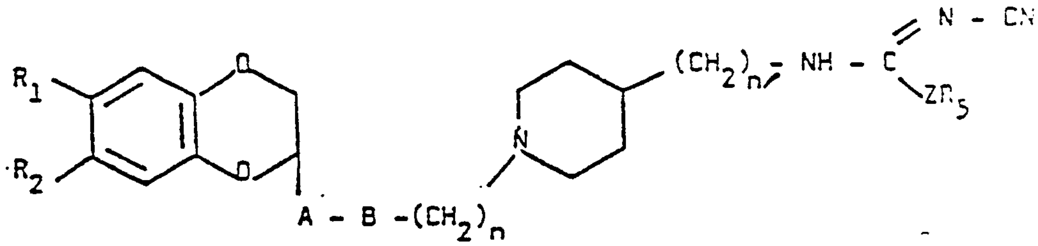 (V)

ou de formule générale $V_A$

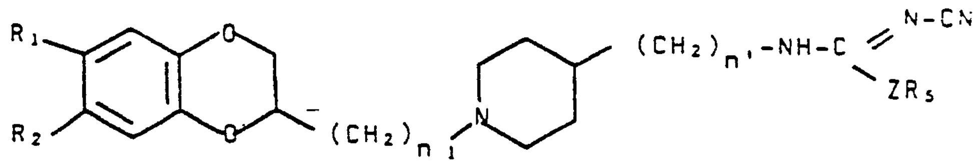 ($V_A$)

dans lesquelles les substituants A, B, $R_1$, $R_2$, $R_5$, n, n' et $n_1$ sont définis comme précédemment et Z est de l'oxygène ou du soufre que l'on fait réagir avec une amine primaire ou secondaire de formule $R_3$ NH $R_4$ pour obtenir la cyanoguanidine de formule générale I

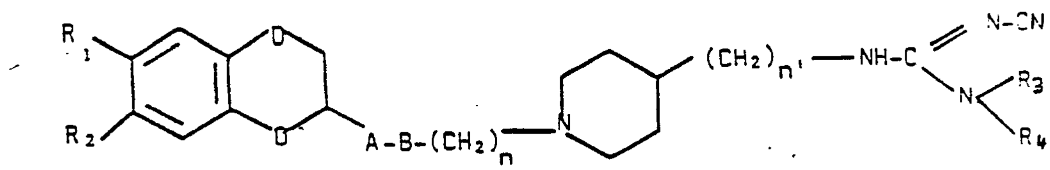 (I)

ou de formule générale $I_A$

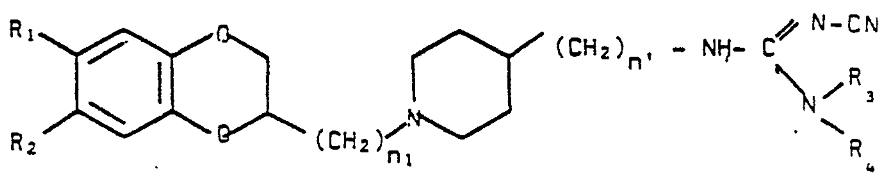 ($I_A$)

dans lesquelles $R_1$, $R_2$, A, B, $R_1$, $R_2$, $R_3$, n et $n_1$ sont définis comme précédemment et $R_4$ est de l'hydrogène ou un radical alcoyle inférieur que l'on peut si désiré, lorsque $R_4$ est de l'hydrogène, acyler par action d'un dérivé fonctionnel d'acide carboxylique pour former une N'-acyl N-cyano-guanidine de formule générale I

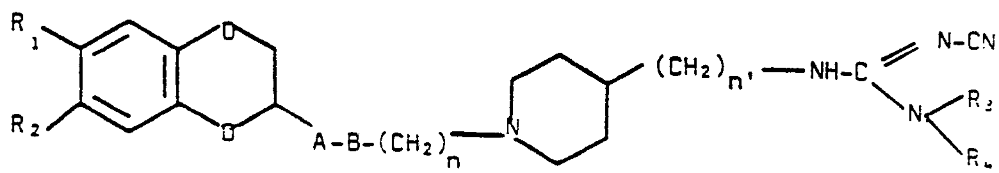 (I)

ou de formule générale $I_A$

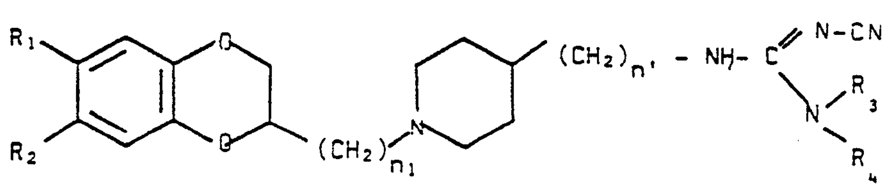 ($I_A$)

dans lesquelles A, B, $R_1$, $R_2$, $R_3$, n, n' et $n_1$ ont les définitions antérieures et $R_4$ le reste acyle d'un acide carboxylique ayant de 1 à 12 atomes de carbone ou bien encore dédoubler par action d'un acide organique optiquement actif en ses somères optiques ou bien encore salifier par addition d'un acide minéral ou organique.

12. En tant que composés intermédiaires de la synthèse, les isothio- ou les iso-urées de formule générale V

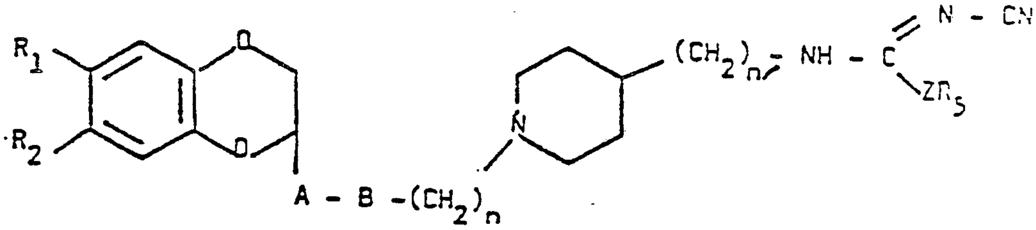

(V)

ou de formule générale $V_A$

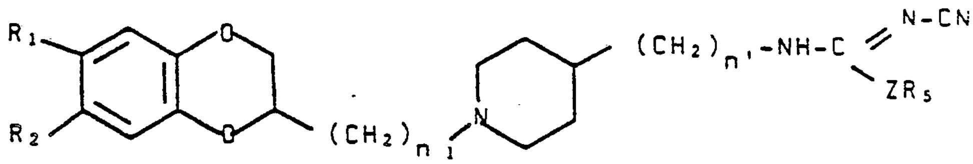

($V_A$)

dans lesquelles la définition des substituants A, B, $R_1$, $R_2$, $R_5$, n, n' et $n^1$ demeure celle fournie précédemment et Z est de l'oxygène ou du soufre.

## Claims

1. The 4-(N-cyano N'-R) guanidinyl piperidines selected from the group consisting of

a) The piperidines of formula I

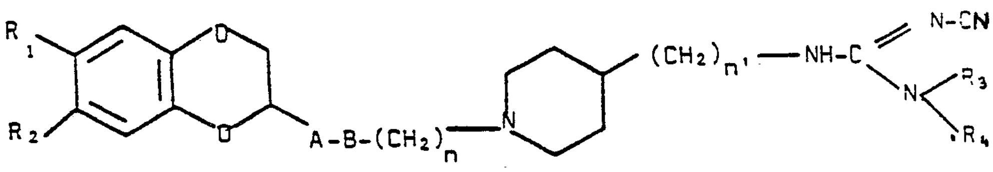

(I)

wherein

A means —$CH_2$— or a simple bond and B is —CHOH— or C = O or A means —CHOH— or C = O and B is a simple bond or —$CH_2$—

n is equal to zero or one

b) The piperidine of formula $I_A$

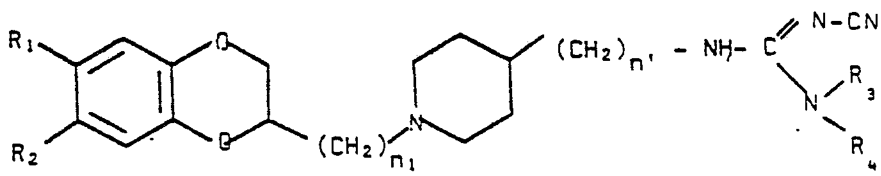

($I_A$)

wherein $n_1$ is an integer from 1 to 3

$R_1$ and $R_2$, the same or different, are a hydrogen, a lower alkyl radical having from 1 to 6 carbon atoms, a lower alkoxy radical having from 1 to 6 carbon atoms, a halogen, or a trifluorométhyl radical

$R_3$ is a lower alkyl radical having from 1 to 6 carbon atoms, a lower alkenyl radical having from 2 to 4 carbon atoms, a lower cycloalkyl having from 3 to 7 carbon atoms or a heterocycloalkyl radical having from 5 to 7 carbon atoms.

$R_4$ is a hydrogen, a lower alkyl radical having from 1 to 6 carbon atoms, or the acyl moiety of an organic carboxylic acid having from 1 to 12 carbon atoms,

or $R_3$ and $R_4$ together are the alkylène chain of a nitrogen — containing heterocycle optionally including a further heteroatom, having from 5 to 7 links

n' is equal to zero or 1

c) and the salts of a compound of formula I or $I_A$ with a mineral or organic acid

2. The acid addition salts of a compound of formula I according to claim 1 with a mineral or organic acid, preferably a therapeutically — compatible acid.

3. The optically — active isomers of the compounds of formula I as well as the diastereo isomers of the compounds of formula I according to claim 1.

4. The piperidines of formula $I_A$ according to claim 1 selected from the group consisting of the piperidines having the formula $I_A$

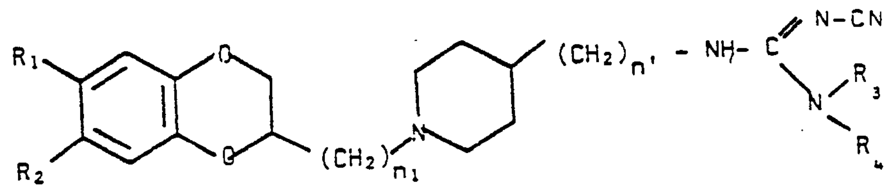

($I_A$)

wherein the substituents $R_1$, $R_2$, $R_3$, $R_4$ and n' have the above — given meanings and the acid addition salts of a compound of formula $I_A$ with a mineral or organic acid

5. The piperidines according to claim 1 having the formula $I_B$, selected from the group consisting of the piperidines of formula $I_B$

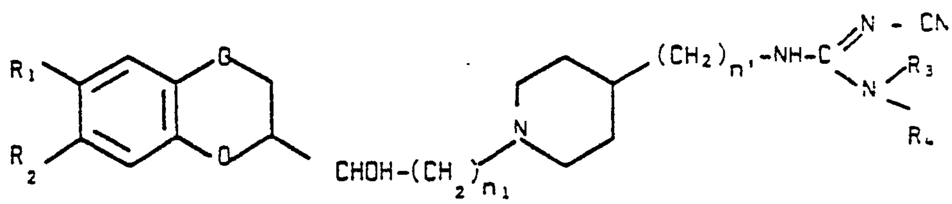

($I_B$)

wherein the substituents $R_1$, $R_2$, $R_3$, $R_4$ and n' have the above — given definitions and $n_1$ is an integer from zero to 2 and the acid addition salts of a compound of formula $I_B$ with a mineral or organic acid.

6. The piperidines according to claim 1 having the formula $I_C$ selected from the group consisting the piperidines of formula $I_C$

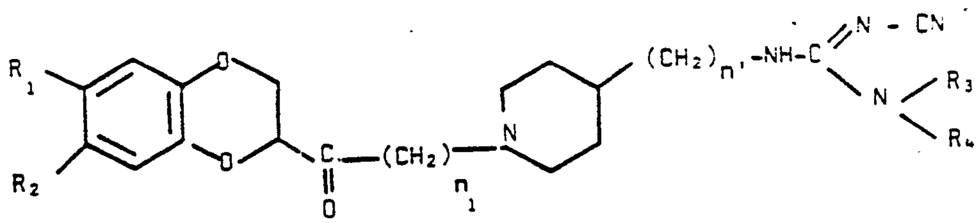

($I_C$)

wherein the substituents $R_1$, $R_2$, $R_3$, $R_4$ and n' have the above — given definitions and $n_1$ is an integer from zero to 2 and the acid addition salts of a compound of formula $I_C$ with a mineral or organic acid.

7. [(2,3-dihydro [4H] 1,4-benzodioxin-2 yl) méthyl] 4-[(N-cyano N'-méthyl) guanidinyl] piperidine according to claim 1

8. 1-[(2,3-dihydro [4H] (1,4-benzodioxin-2 yl) méthyl] 4-[(N-cyano N'-méthyl) guanidinyl méthyl] piperidine according to claim 1.

9. 1-[2,3-dihydro [4H] (1,4-benzodioxin-2 yl) méthyl] 4-(N-cyano N'-allyl) guanidinyl méthyl] piperidine according to claim 1.

10. The pharmaceutical compositions incorporating as active ingredient at least one compound according to any of claims 1 to 9 in admixture or conjunction with an inert, non-toxic, pharmaceutically — acceptable carrier or vehicle.

11. A process for preparing the compounds of formula I according to claim 1 characterized in that a 4-aminopiperidine of formula II

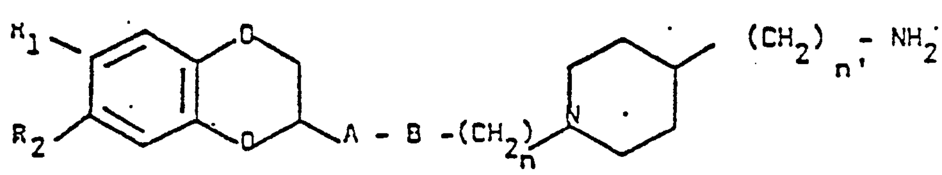

(II)

or of formula $II_A$

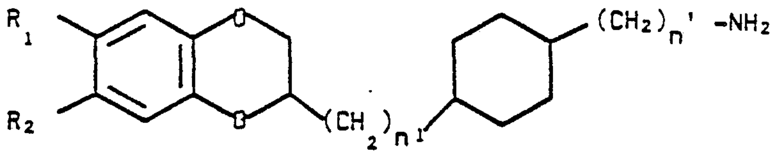

(II$_A$)

wherein $R_1$, $R_2$, A, B, $n_1$ and n' are defined as above is reacted with a cyano — iminating reagent selected from the group consisting of the alkyl cyanoimino isodithiocarbonates of the formula III

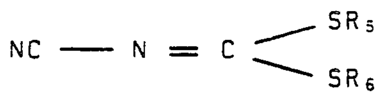

(III)

wherein $R_5$ and $R_6$ are a lower alkyl radical and — the mixed alkyl cyanoimino isothiocarbonates of the formula IV

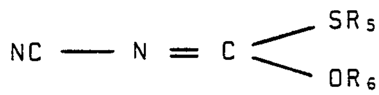

(IV)

wherein $R_5$ and $R_6$ are defined as above — given to produce an Isothio — Urea or an Iso Urea of the formula V

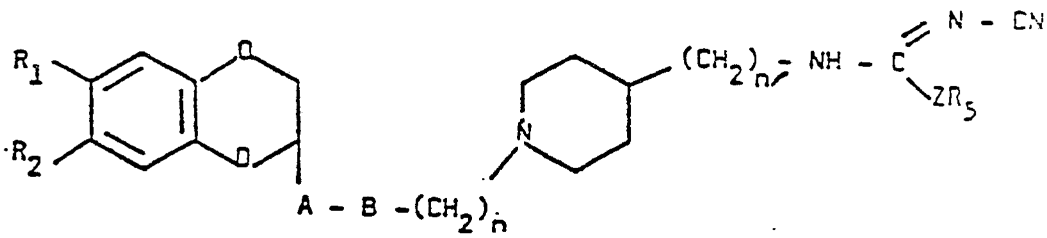

(V)

or of the formula $V_A$

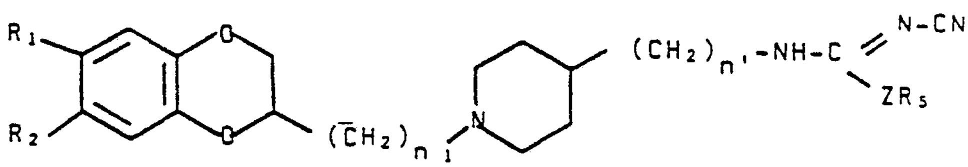

($V_A$)

wherein the substituents A, B, $R_1$, $R_2$, $R_5$, n, n' and $n_1$ have the above — given definitions and Z is an oxygen or a sulphur and reacting the latter with a primary or a secundary amine of the formula

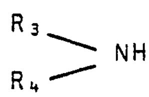

to obtain a cyanoguanidine of formula I

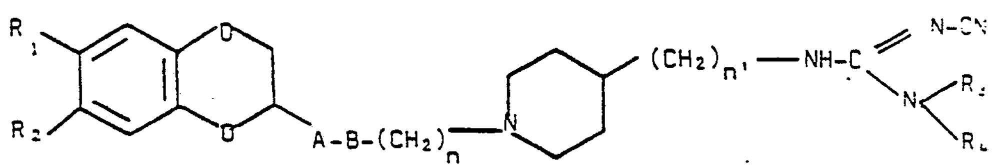

(I)

or a cyanoguanidine of formulat $I_A$

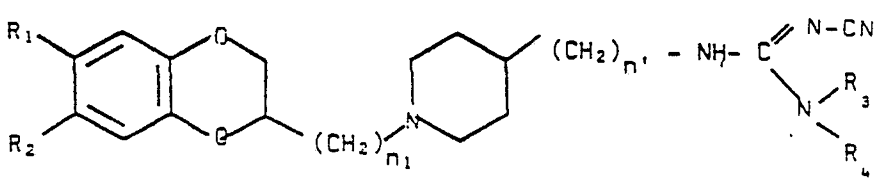

($I_A$)

wherein A, B, $R_1$, $R_2$, $R_3$, n' and $n_1$ have the above given definitions and $R_4$ is hydrogen or a lower alkyl radical which may, if desired, when $R_4$ is hydrogen, be acylated by means of a functional derivative of a carboxylic acid to produce a N'-acyl N-cyano guanidine of formula I

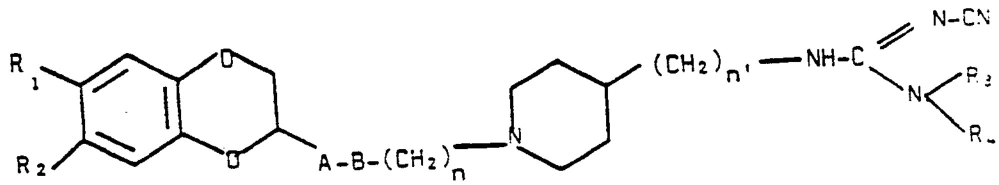

(I)

or a N'-acyl N-cyanoguanidine of formula $I_A$

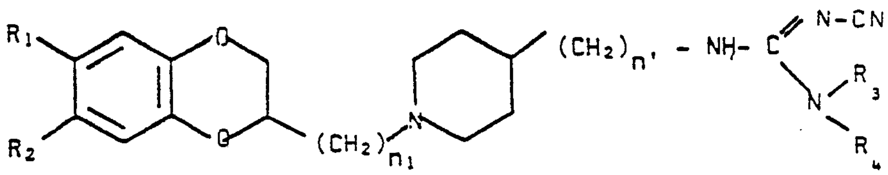

($I_A$)

wherein A, B, $R_1$, $R_2$, $R_3$, n, n' and $n_1$ have the above — given definitions and $R_4$ is the acyl moiety of a carboxylic acid having from 1 to 12 carbon atoms or be resolved into their optical — isomers by means of an optically — active organic acid or be salified by adding a mineral or organic acid.

12. As intermediate products in the synthesis, the Iso Thio — and the Iso Ureas of formula V

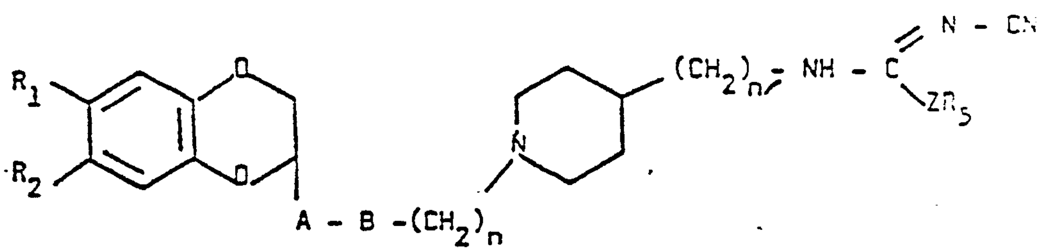

(V)

and the Isothio — and the Iso Ureas of formula $V_A$

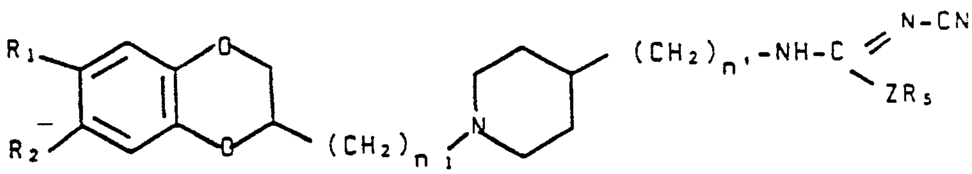

($V_A$)

wherein the definitions of the substituents A, B, $R_1$, $R_2$, n, n' and $n_1$ are the same as previously given and Z is an oxygen or a sulphur.

**Patentansprüche**

1. Die 4-(N-cyan N'-R) guanidinyl piperidinen die aus die Gruppe gewählt sind die

a) die Piperidinen der Formel I

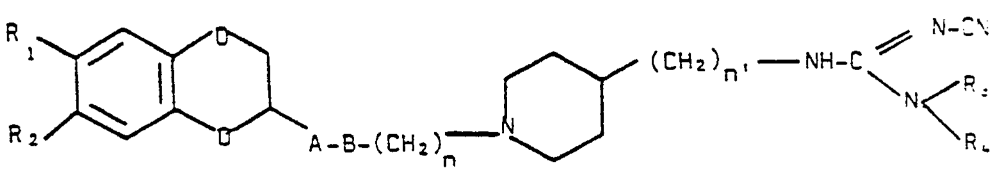

(I)

in der

A —$CH_2$— oder eine einfache Bund ist und B —CHOH— oder C = O ist oder A —CHOH— oder C = O bedeutet und B eine einfache

Bund oder —$CH_2$— ist und

n für die Zahlen null oder 1 steht

b) die Piperidinen der Formel $I_A$

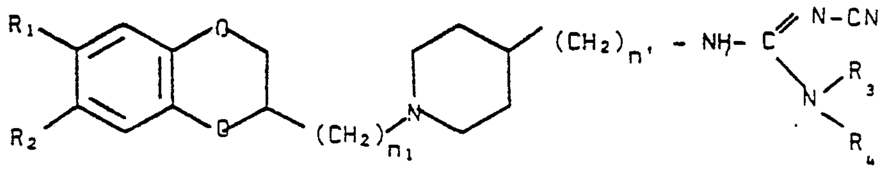 ($I_A$)

in der $n_1$ eine ganze Zahl von 1 bis 3 ist

$R_1$ und $R_2$, gleich oder verschieden sind, Wasserstoff, eine 1 bis 6 Kohlenstoff-atomen enthaltènde niedrig·Alkyl Gruppe, eine 1 bis 6 Kohlen-stoffatomen-enthaltende niedrig-Alkoxy Gruppe, ein Halogen, oder eine Trifluoromethyl Gruppe sind.

$R_3$, eine 1 bis 6 Kohlenstoff-atomen enthaltende niedrig-Alkyl Gruppe, eine 2 bis 4 Kohlenstoffatomen niedrig-Alkenylgruppe, eine 3 bis 7 Kohlenstoff-atomen enthaltende Cycloalkyl Gruppe, oder eine 5 bis 7 Kohlenstoffatomen enthaltende Heterocycloalkyl Gruppe ist.

$R_4$ Wasserstoff, eine 1 bis 6 Kohlenstoffatomen-enthaltende niedrig Alkyl Gruppe oder der Acyl-rest mit 1 bis 12 Kohlenstoffatomen einer organischen Carbonsäure ist.

oder $R_3$ und $R_4$ zusammen die Alkylen Kette eine 5 bis 7 gliedrigen Stickstoff enthaltende Heterocyclus welcher gegebenenfalls eine weitere Heteroatom enthält, sind

n' null oder 1 ist und

c) die Salze einer Verbindung der allgemeinen Formel I oder $I_A$ mit einer anorganischen oder organischen Säure,

enthält.

2. Die Salze einer Verbindung der allgemeinen Formel I nach Anspruch 1, mit einer anorganischen oder organischen Säure, vorzuglich eine therapeutisch-verträgliche Säure.

3. Die optisch-aktive Isomere der Verbindung der allgemeinen Formel I und die Diastereoisomere der Verbindungen der allgemeinen Formel I nach Anspruch 1.

4. Die Piperidinen der allgemeinen Formel $I_A$ nach Anspruch 1 aus die Gruppe gewählt die die Piperidinen mit der allgemeinen Formel $I_A$

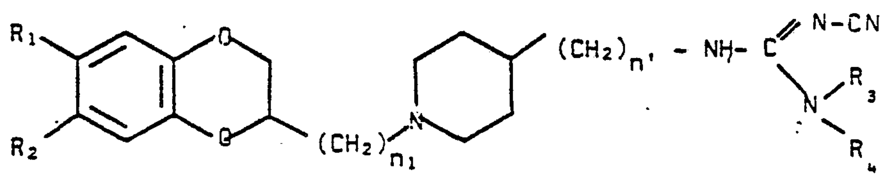 ($I_A$)

worin die Substituenten $R_1$, $R_2$, $R_3$, $R_4$ und n' die oben angegebene Bedeutungen haben und

die Salze einer Verbindung der allgemeinen Formel $I_A$ mit einer anorganischen oder organischen Säure

enthält.

5. Die Piperidinen der allgemeinen Formel $I_B$ nach Anspruch 1 aus die Gruppe gewählt die die Piperidinen der allgemeinen Formel $I_B$

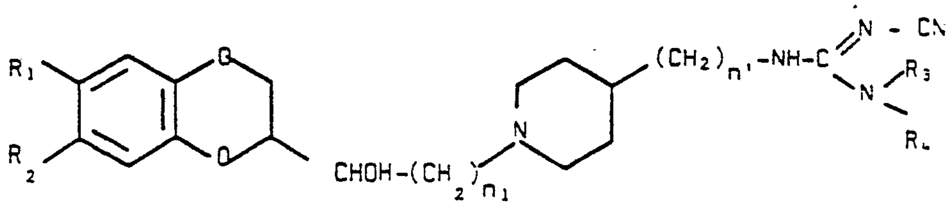 ($I_B$)

in der die Substituenten $R_1$, $R_2$, $R_3$, $R_4$ und n' die selbe Bedeutungen als oben angegeben haben und $n_1$ eine ganze Zahl von null bis 2 ist und

die Salze einer Verbindung der allgemeinen Formel $I_B$ mit einer anorganischen oder organischen Säure

enthält.

6. Die Piperidinen der allgemeinen Formel $I_C$ nach Anspruch 1 aus die Gruppe gewählt die die Piperidinen der Formel $I_C$

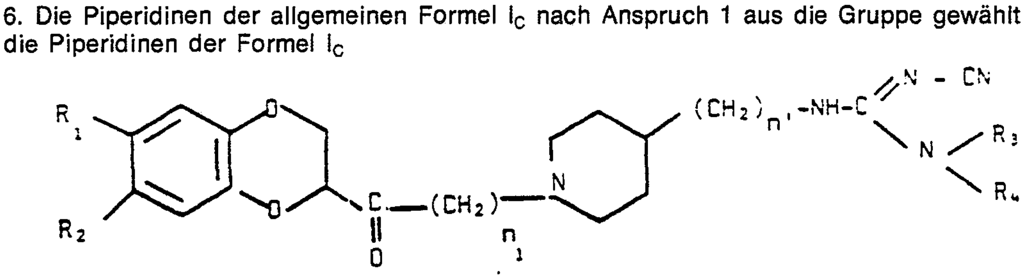 ($I_C$)

in welcher die Substituenten $R_1$, $R_2$, $R_3$, $R_4$ und n' die selbe Bedeutung als oben angegeben, haben und $n_1$ eine ganze Zahl von null bis 2 ist und

die Salze einer Verbindungen der allgemeinen Formel $I_C$ mit einer anorganischen oder organischen Säure

enthält.

7. 1-[(2,3-Dihydro [4H] 1,4-benzodioxin-2 yl) methyl] 4-[(N-cyan N'-Methyl) guanidinyl methyl] piperidin nach Anspruch 1

8. 1-[(2,3-Dihydro [4H] 1,4-benzodioxin-2 yl) methyl] 4-[(N-cyan N'-allyl) guanidinyl methyl] piperidin nach Anspruch 1

9. 1-[(2,3-Dihydro [4H] 1,4-benzodioxin-2 yl) methyl] 4-[(N-cyan N'-allyl) guanidinyl methyl] piperidin nach Anspruch 1

10. Pharmazeutische Zusammensetzungen, die als Wirkstoff mindenstens eine Verbindung nach etwaige Anspruch 1 bis 9 enthält in Gemisch oder in Vereinigung mit einem inerten, nicht-giftig pharmazeutisch-verträgliche Träger oder Vehikel

11. Verfahren fur die Herstellung der Verbindungen der allgemeinen Formel I nach Anspruch 1 dadurch gekennzeichnet daß man eine 4-amino piperidin der allgemeinen Formel II

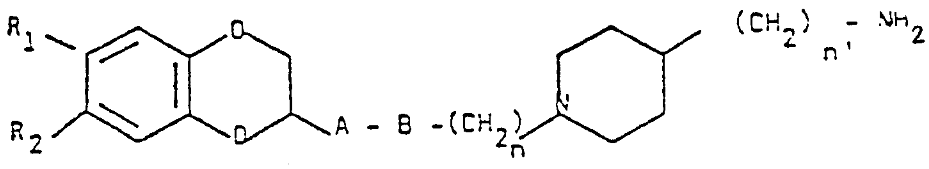

(II)

oder der allgemeinen Formel $II_A$

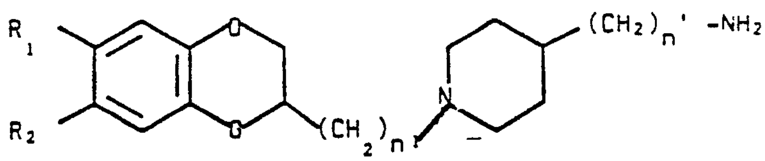

($II_A$)

in welchen $R_1$, $R_2$, A, B, $n_1$ und n' die oben angegebene Bedeutungen besitzen mit einem für Cyan-Iminierung Reagenz aus die Gruppe gewählt die die Alkyl Cyaniminoisodithiocarbonsäure der allgemeinen Formel III

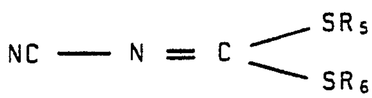

(III)

worin $R_5$ und $R_6$ niedrig Alkyl gruppe sind und — die gemischte Alkyl Cyaniminoisothiocarbonsäure der allgemeinen Formel IV

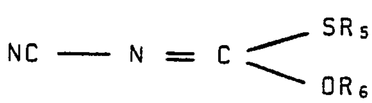

(IV)

worin $R_5$ und $R_6$ wie vorher definiert sind, enthält, umsetzt, um das Isothioharnstoff oder Isoharnstoff der allgemeinen Formel V

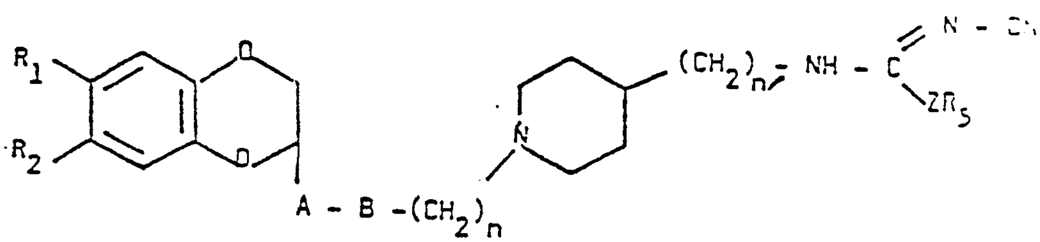

(V)

oder der allgemeinen Formel $V_a$

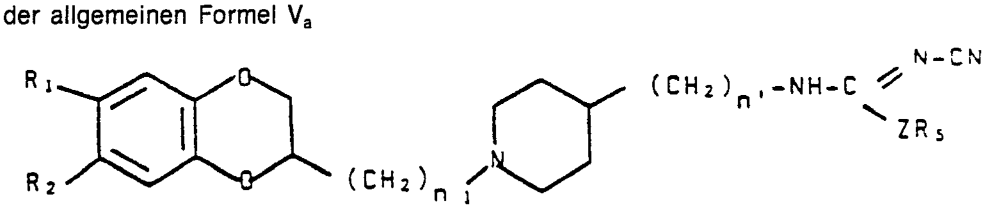

($V_A$)

zu bilden in welchen die Substituenten A, B, $R_1$, $R_2$, $R_3$, n, n' und $n_1$ wie vorher definiert sind und Z ein Sauerstoff oder Schwefelatom ist, die mit einer primären- oder sekundären Amin der Formel

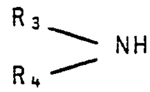

umwandeln lässt um die Cyanoguanidin der allgemeinen Formel I

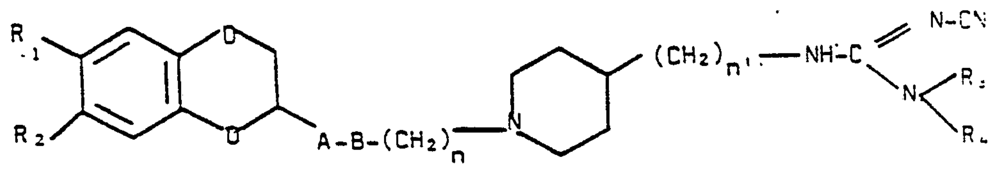

(I)

oder der allgemeinen Formel $I_A$

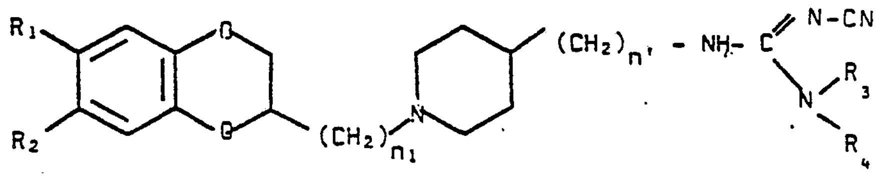

($I_A$)

worin A, B, $R_1$, $R_2$, $R_3$, n und $n_1$ wie oben definiert sind und R Wasserstoff oder eine niedrig-Alkyl Gruppe ist zu herstellen, das gewünchenfalls, wenn $R_4$ ein Wasserstoff ist, mittels einem funktionnell Derivat einer Carbonsäure acyliert kann um ein N'-acyl N'-cyanguanidin der allgemeinen Formel I

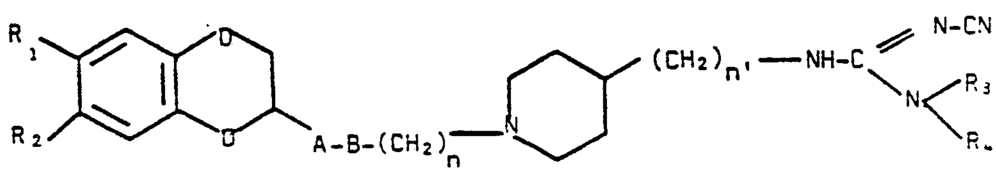

(I)

oder der allgemeinen Formel $I_A$

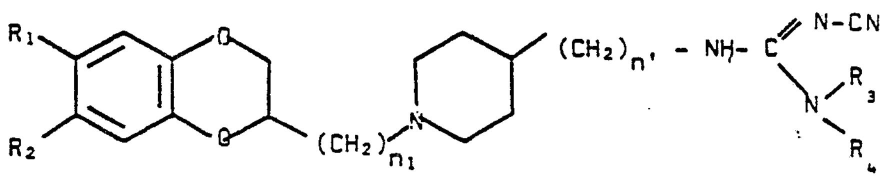

($I_A$)

worin A, B, $R_1$, $R_2$, $R_3$, n, n' und $n_1$ die oben-angegebene Bedeutungen besitzen und R den Acyl Rest einer Carbonsäure mit 1 bis 12 Kohenstoffatomen ist oder in seiner optischaktive Isomeren mittels einer optisch-aktive organische Säure spalten lässt oder durch Zugabe einer anorganischen oder organischen Säure in eine Salz umwandeln lässt.

12. Als Zwischenprodukte in der Synthesis, die Isothio- oder Isoharnstoffe der allgemeinen Formel V

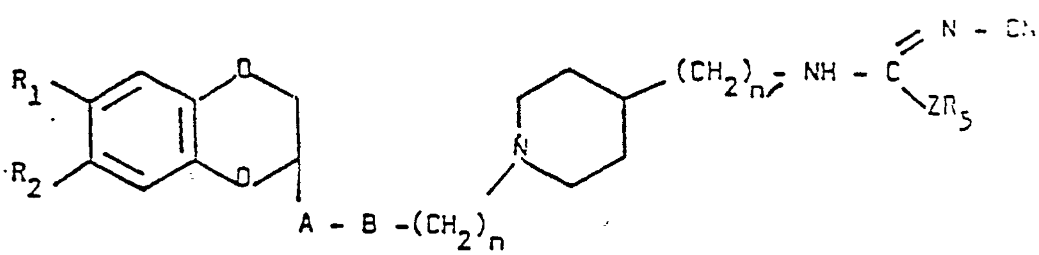

(V)

oder der allgemeinen Formel Va

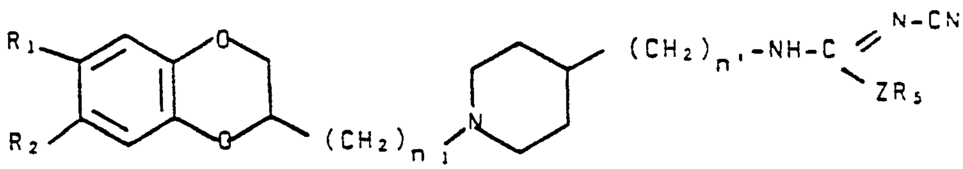

$(V_A)$

worin die Bedeutungen der Substituenten A, B, $R_1$, $R_2$, $R_5$, n, n' und $n_1$ die selbe als vorher angegeben, bleiben und Z ein Sauerstoff oder ein Schwefelatom ist.